# EUROPÄISCHE PATENTANMELDUNG

(11) **EP 2 263 641 A2**
(43) Veröffentlichungstag der Anmeldung: **22.12.2010**
(21) Anmeldenummer: 10158189.0
(22) Anmeldetag: 29.03.2010
(51) Int. Cl.: A61K 8/34, A61K 8/64, A61K 8/66, A61K 8/67, A61Q 19/08, A61K 8/36

(54) **Antifalten-Kosmetikum mit Antioxidantien**

(30) Priorität: 18.06.2009 DE 102009027024
(71) Anmelder: Henkel AG & Co. KGaA, 40589 Düsseldorf (DE)
(72) Erfinder: Janßen, Frank, 41470, Neuss (DE); Dickhof, Susanne, 41748, Viersen (DE); Emond, Eliane, 40470, Düsseldorf (DE)

(57) **Zusammenfassung**

Gegenstand der vorliegenden Anmeldung sind kosmetische Zusammensetzungen, die das Tetrapeptid Lysin-Asparaginsäure-Isoleucin-Citrullin Lys-Asp-lle-Cit-NH₂ und mindestens ein Antioxidans enthalten, das ausgewählt ist aus Carotinoiden, insbesondere Lycopin und Astaxanthin, Dihydroquercetin, 3,4-Dihydro-7-methoxy-2,2-dimethyl-2H-1-benzopyran-6-ol, Vitis vinifera-Polyphenolen, Ubichinonen und/oder Ubichinolen, Superoxid-Dismutasen und Ellagsäure, sowie Mischungen dieser Antioxidantien.

## Beschreibung

Gegenstand der vorliegenden Anmeldung sind kosmetische Zusammensetzungen, die das Tetrapeptid Lysin-Asparaginsäure-Isoleucin-Citrullin Lys-Asp-Ile-Cit-NH₂ und mindestens ein Antioxidans enthalten, das ausgewählt ist aus Carotinoiden, insbesondere aus den Carotinoiden Lycopin und Astaxanthin, weiterhin ausgewählt aus Dihydroquercetin, 3,4-Dihydro-7-methoxy-2,2-dimethyl-2H-1-benzopyran-6-ol, Vitis vinifera-Polyphenolen, Ubichinonen und/oder Ubichinolen, Superoxid-Dismutasen und Ellagsäure, sowie Mischungen dieser Antioxidantien.

Die Hautalterung ist geprägt durch eine Reihe an strukturellen Veränderungen. Es kommt zur Reduzierung der Epidermisdicke, zur Abflachung der dermalen-epidermalen Verbindungslinie, zum Abbau der Matrixproteine und zur Glycosylierung von Matrixproteinen. Neben der genetisch vorgegebenen chronologischen oder intrinsischen Hautalterung werden die Alterungsprozesse durch extrinsische Faktoren beschleunigt. Zu diesen extrinsischen Faktoren zählt insbesondere die Belastung der Haut mit freien Radikalen, der auch als "oxidativer Stress" bezeichnet wird. Unter oxidativem Stress versteht man eine Störung der Balance zwischen Oxidantien und Antioxidantien in der lebenden Zelle zugunsten der Oxidantien, wobei es durch chemische Reaktionen zu Schädigungen von Proteinen, DNA und Lipiden kommt. Die eintretenden Zellschädigungen können zur Bildung von Krebs und zum Zelltod führen. Oxidantien, die oxidativen Stress hervorrufen, sind üblicherweise reaktive Sauerstoff-Spezies (z. B. Hyperoxid, Hydroxyl-Radikale, Lipid-Hydroperoxide, Singulett-Sauerstoff, Wasserstoffperoxid und Ozon) sowie reaktive Stickstoff-Spezies (z. B. Nitroxyl-Radikale und Stickstoffoxide). Der oxidative Stress kann z. B. durch Strahlungseinwirkung induziert werden und spielt eine Rolle bei der Entstehung einer Reihe akuter und chronischer Erkrankungen (z. B. Entzündungen verschiedener Ursache, Arteriosklerose und Mikroangiopathien). Zur Abwehr des oxidativen Stresses unterhält der Körper ein Reservoir unterschiedlicher reduzierter Verbindungen (Antioxidantien) wie L-Ascorbinsäure, Dihydroliponsäure, Harnsäure, Glutathion oder alpha-Tocopherol. Außerdem vermeidet er mit Hilfe verschiedener Enzym-Aktivitäten (z. B. Glutathion-Peroxidase und Katalase) das Auftreten reaktiver Radikale.

### Stand der Technik

Die Auswirkungen des oxidativen Stresses auf die Alterung der Haut sind bekannt. Viele kosmetische Zusammensetzungen enthalten mindestens ein Antioxidans, um - neben der notwendigen Stabilisierung enthaltener Öle und Fette vor autoxidativem Verderb - der Hautalterung entgegenzuwirken. US 4144325 und US 4248861 sowie zahlreiche weitere Dokumente offenbaren kosmetische Zusammensetzungen, die Vitamin E, eine Substanz mit bekannter antioxidativer Wirkung, enthalten. JP 58180410 A und DE 3309850 A offenbaren kosmetische Zusammensetzungen, die das bekannte Antioxidans Coenzym Q-10 enthalten, um den Hautzellmetabolismus zu aktivieren und die Oxidation zu unterdrücken und so Hautschäden durch UV-Strahlen und der Hautalterung vorzubeugen. EP 1002533 A1 offenbart die Verwendung von 2,2-Dimethylchromanen und -chromenen als Antioxidantien in Zusammensetzungen zur topischen Verabreichung. EP 1430882 A2 offenbart kosmetische Zusammensetzungen, die 6,7-disubstituierte 2,2-Dialkylchromane oder -chromene in synergistischer Kombination mit weiteren bekannten Antioxidantien enthalten. EP 1174140 A1 offenbart Chromanolglycoside, insbesondere 2,5,7,8-Tetramethylchroman-6-ol, das in 2-Position mit einem Glucopyranosylmethyl-, Galactopyranosylmethyl-, Fructofuranosylmethyl- oder Mannopyranosylmethyl-Rest substituiert ist, als Wirkstoff, der effektiv freie Radikale eliminiert.

Das EU-Projekt "Cellage" (Project Reference QLK3-CT-2002-71628) fand bei der Prüfung bekannter antioxidativer Substanzen gegen die Hautalterung (Vitamin A, Vitamin E) u. a. heraus, dass deren positive Wirkung auf die Alterungsvorgänge in Hautzellkulturen konzentrationsabhängig ist und dass sie bei zu hoher Konzentration auch toxisch wirken können.

### Aufgabe

Aufgabe der vorliegenden Erfindung war es, kosmetische Zusammensetzungen mit verbesserter antioxidativer Wirkung bereitzustellen, die gegen die Hautalterung wirksam sind und gleichzeitig mit einer minimalen Menge an Antioxidantien eine maximale antioxidative Wirkung erzielen und sich somit in wirksamer und hautverträglicher Weise zur kosmetischen Behandlung und Prävention der Hautalterung eignen.

In überraschender Weise wurde gefunden, dass kosmetische Produkte, die das Tetrapeptid Lysin-Asparaginsäure-Isoleucin-Citrullin Lys-Asp-Ile-Cit-NH₂ in Kombination mit ausgewählten Antioxidanzien enthalten, eine synergistische Steigerung der antioxidativen Wirkung aufzeigen und damit besonders geeignet erscheinen, in wirksamer und hautverträglicher Weise die extrinsische Hautalterung kosmetisch zu behandeln. Die mit einer erfindungsgemäßen Zusammensetzung behandelte Haut zeigte ein verbessertes Hautbild und verbesserte Ausstrahlung der Haut. Die untersuchten Zeichen der Hautalterung wiesen ein reduziertes Ausmaß auf.

Ein erster Gegenstand der vorliegenden Anmeldung ist eine kosmetische Zusammensetzung, die das Tetrapeptid Lysin-Asparaginsäure-Isoleucin-Citrullin Lys-Asp-Ile-Cit-NH₂ in Kombination mit mindestens einem Antioxidans enthält, das ausgewählt ist aus
- Carotinoiden, insbesondere Lycopin und Astaxanthin,
- Dihydroquercetin,
- 3,4-Dihydro-7-methoxy-2,2-dimethyl-2H-1-benzopyran-6-ol,
- Vitis vinifera-Polyphenolen,
- Ubichinonen und/oder Ubichinolen,
- Superoxid-Dismutasen,
- Ellagsäure und/oder Ellagaten,
- sowie Mischungen dieser Antioxidantien.

Erfindungsgemäß bevorzugte kosmetische Zusammensetzungen enthalten - bezogen auf ihr Gewicht - das Tetrapeptid Lysin-Asparaginsäure-Isoleucin-Citrullin Lys-Asp-Ile-Cit-NH₂ in einer Menge von 0,00001 - 0,001 Gew.-%, bevorzugt 0,0001 - 0,0008 Gew.-%, besonders bevorzugt 0,0002 - 0,0006 Gew.-%, außerordentlich bevorzugt 0,0003 - 0,0004 Gew.-%.

Weitere erfindungsgemäß bevorzugte kosmetische Zusammensetzungen enthalten - bezogen auf ihr Gewicht - das mindestens eine Antioxidans, das ausgewählt ist aus Carotinoiden, Dihydroquercetin, 3,4-Dihydro-7-methoxy-2,2-dimethyl-2H-1-benzopyran-6-ol, Vitis vinifera-Polyphenolen, Ubichinonen und/oder Ubichinolen, Superoxid-Dismutasen, Ellagsäure und/oder Ellagaten, sowie Mischungen dieser Antioxidantien, in einer Gesamtmenge von 0,001 - 2 Gew.-%, bevorzugt 0,005 - 1 Gew.-%, besonders bevorzugt 0,01 - 0,5 Gew.-%, außerordentlich bevorzugt 0,05 - 0,2 Gew.-%.

Weitere erfindungsgemäß bevorzugte kosmetische Zusammensetzungen enthalten - bezogen auf ihr Gewicht - das Tetrapeptid Lysin-Asparaginsäure-Isoleucin-Citrullin Lys-Asp-Ile-Cit-NH₂ in einer Menge von 0,00001 - 0,001 Gew.-%, und das mindestens eine Antioxidans, das ausgewählt ist aus Carotinoiden, Dihydroquercetin, 3,4-Dihydro-7-methoxy-2,2-dimethyl-2H-1-benzopyran-6-ol, Vitis vinifera-Polyphenolen, Ubichinonen und/oder Ubichinolen, Superoxid-Dismutasen, Ellagsäure und/oder Ellagaten, sowie Mischungen dieser Antioxidantien, in einer Gesamtmenge von 0,001 - 2 Gew.-%.

Zu den erfindungsgemäß bevorzugten Carotinoiden zählen
- die Carotine, bevorzugt α-Carotin, β-Carotin und Lycopin sowie
- die Xanthophylle, bevorzugt Astaxanthin, Lutein, Zeaxanthin, Capsanthin, Violaxanthin, Crocetin und Bixin.

Bis heute sind über 600 verschiedene natürlich vorkommende Carotinoide bekannt. Sie sind in Bakterien (z. B. Rhodopin, Spirilloxanthin, Sarcinaxanthin), Cyanobakterien (z. B. Myxoxanthin, Oscillaxanthin, beta-Carotin), Algen (z. B. Fucoxanthin, Violaxanthin, Diatoxanthin), Pilzen (Canthaxanthin, Aleuriaxanthin) und in höheren Pflanzen (z. B. alpha-Carotin, beta-Carotin, gamma-Carotin, Lycopin, Capsanthin, Capsorubin, Lutein, Zeaxanthin, Crocetin, Bixin, Citranaxanthin, Cryptoxanthin, Flavoxanthin, Neoxanthin, Rhodoxanthin, Rubixanthin, Violaxanthin) in den Plastiden von Blättern, Früchten, Sprossen, Wurzeln, Pollen und Samen angereichert. Xanthophylle ist der Gruppenname für Hydroxy-, Epoxy- und Oxo-Derivate von Carotinoiden (= Tetraterpenen mit einem C₄₀-Grundgerüst). Beispiele für erfindungsgemäß geeignete Verbindungen sind z. B. Canthaxanthin, Citranaxanthin, Flavoxanthin, Rhodoxanthin, Rubixanthin, Zeaxanthin, Lutein, Cryptoxanthin, Violaxanthin und insbesondere Astaxanthin. Die natürlich vorkommenden Xanthophylle sind meist *all-trans-*Verbindungen. Erfindungsgemäß besonders bevorzugt sind natürlich vorkommende Xanthophylle.

Weitere erfindungsgemäß bevorzugte Zusammensetzungen enthalten - bezogen auf ihr Gewicht
- das mindestens eine Carotinoid in einer Gesamtmenge von 0,001 - 2 Gew.-%, bevorzugt 0,005
- 1 Gew.-%, besonders bevorzugt 0,01 - 0,5 Gew.-%, außerordentlich bevorzugt 0,05 - 0,2 Gew.-%.

Weitere erfindungsgemäß bevorzugte Zusammensetzungen enthalten - bezogen auf ihr Gewicht
- das Tetrapeptid Lysin-Asparaginsäure-Isoleucin-Citrullin Lys-Asp-Ile-Cit-NH₂ in einer Menge von 0,00001 - 0,001 Gew.-% und das mindestens eine Carotinoid in einer Gesamtmenge von 0,001 - 2 Gew.-%, bevorzugt 0,005 - 1 Gew.-%, besonders bevorzugt 0,01 - 0,5 Gew.-%, außerordentlich bevorzugt 0,05 - 0,2 Gew.-%.

Weitere erfindungsgemäß bevorzugte Zusammensetzungen enthalten - bezogen auf ihr Gewicht
- das Tetrapeptid Lysin-Asparaginsäure-Isoleucin-Citrullin Lys-Asp-Ile-Cit-NH₂ in einer Menge von 0,00001 - 0,001 Gew.-% und Lycopin in einer Gesamtmenge von 0,001 - 2 Gew.-%, bevorzugt 0,005 - 1 Gew.-%, besonders bevorzugt 0,01 - 0,5 Gew.-%, außerordentlich bevorzugt 0,05
- 0,2 Gew.-%.

Weitere erfindungsgemäß bevorzugte Zusammensetzungen enthalten - bezogen auf ihr Gewicht
- das Tetrapeptid Lysin-Asparaginsäure-Isoleucin-Citrullin Lys-Asp-Ile-Cit-NH₂ in einer Menge von 0,00001 - 0,001 Gew.-% und Lutein in einer Gesamtmenge von 0,001 - 2 Gew.-%, bevorzugt 0,005 - 1 Gew.-%, besonders bevorzugt 0,01 - 0,5 Gew.-%, außerordentlich bevorzugt 0,05
- 0,2 Gew.-%.

Weitere erfindungsgemäß bevorzugte Zusammensetzungen enthalten - bezogen auf ihr Gewicht
- das Tetrapeptid Lysin-Asparaginsäure-Isoleucin-Citrullin Lys-Asp-Ile-Cit-NH₂ in einer Menge von 0,00001 - 0,001 Gew.-% und Astaxanthin in einer Gesamtmenge von 0,001 - 2 Gew.-%, bevorzugt 0,005 - 1 Gew.-%, besonders bevorzugt 0,01 - 0,5 Gew.-%, außerordentlich bevorzugt 0,05 - 0,2 Gew.-%.

Weitere erfindungsgemäß bevorzugte Zusammensetzungen sind **dadurch gekennzeichnet, dass** sie das Tetrapeptid Lysin-Asparaginsäure-Isoleucin-Citrullin Lys-Asp-Ile-Cit-NH₂ und das Antioxidans Dihydroquercetin (3,3',4',5,7-Pentahydroxyflavanon, C₁₅H₁₂O₇, Molmasse 304,3 g/mol), das auch unter der Bezeichung Taxifolin bekannt ist, enthalten. Besonders bevorzugt ist das Isomer (2R,3R)-Dihydroquercetin.

Weitere erfindungsgemäß bevorzugte Zusammensetzungen enthalten - bezogen auf ihr Gewicht
- Dihydroquercetin in einer Gesamtmenge von 0,001 - 2 Gew.-%, bevorzugt 0,005 - 1 Gew.-%, besonders bevorzugt 0,01 - 0,5 Gew.-%, außerordentlich bevorzugt 0,05 - 0,2 Gew.-%.

Weitere erfindungsgemäß bevorzugte Zusammensetzungen enthalten - bezogen auf ihr Gewicht
- das Tetrapeptid Lysin-Asparaginsäure-Isoleucin-Citrullin Lys-Asp-Ile-Cit-NH₂ in einer Menge von 0,00001 - 0,001 Gew.-% und Dihydroquercetin in einer Gesamtmenge von 0,001 - 2 Gew.-%, bevorzugt 0,005 - 1 Gew.-%, besonders bevorzugt 0,01 - 0,5 Gew.-%, außerordentlich bevorzugt 0,05 - 0,2 Gew.-%.

Weitere erfindungsgemäß bevorzugte Zusammensetzungen sind **dadurch gekennzeichnet, dass** sie das Tetrapeptid Lysin-Asparaginsäure-Isoleucin-Citrullin Lys-Asp-Ile-Cit-NH₂ und das Antioxidans 3,4-Dihydro-7-methoxy-2,2-dimethyl-2H-1-benzopyran-6-ol enthalten. 3,4-Dihydro-7-methoxy-2,2-dimethyl-2H-1-benzopyran-6-ol ist ein 6,7-disubstituiertes 2,2-Dialkylchroman der allgemeinen Formel (I), 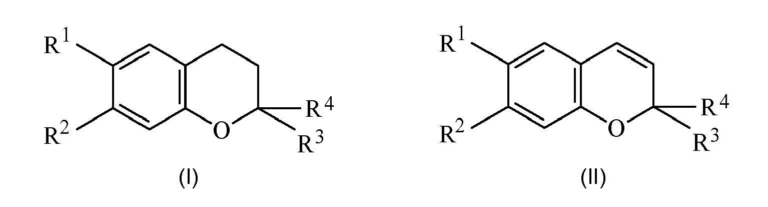 wobei R¹ eine OH-Gruppe, R² eine Methoxy-Gruppe und R³ und R⁴ Methylgruppen darstellen. Die allgemeine Formel (I) stellt die reduzierte, antioxidativ wirksame Form dar, während die Formel (II) die oxidierte Form darstellt.
Weitere erfindungsgemäß bevorzugte Zusammensetzungen enthalten - bezogen auf ihr Gewicht
- 3,4-Dihydro-7-methoxy-2,2-dimethyl-2H-1-benzopyran-6-ol in einer Gesamtmenge von 0,001 - 2 Gew.-%, bevorzugt 0,005 - 1 Gew.-%, besonders bevorzugt 0,01 - 0,5 Gew.-%, außerordentlich bevorzugt 0,05 - 0,2 Gew.-%.
Weitere erfindungsgemäß bevorzugte Zusammensetzungen enthalten - bezogen auf ihr Gewicht
- das Tetrapeptid Lysin-Asparaginsäure-Isoleucin-Citrullin Lys-Asp-Ile-Cit-NH₂ in einer Menge von 0,00001 - 0,001 Gew.-% und 3,4-Dihydro-7-methoxy-2,2-dimethyl-2H-1-benzopyran-6-ol in einer Gesamtmenge von 0,001 - 2 Gew.-%, bevorzugt 0,005 - 1 Gew.-%, besonders bevorzugt 0,01 - 0,5 Gew.-%, außerordentlich bevorzugt 0,05 - 0,2 Gew.-%.

In einer weiteren bevorzugten Ausführungsform sind die erfindungsgemäß in ihrer Wirkung gesteigerten Antioxidantien aus Vitis vinifera-Polyphenolen ausgewählt.
Unter Polyphenolen sind erfindungsgemäß aromatische Verbindungen zu verstehen, die mindestens zwei phenolische Hydroxy-Gruppen im Molekül enthalten. Hierzu zählen die drei Dihydroxybenzole Brenzcatechin, Resorcin und Hydrochinon, weiterhin Phloroglucin, Pyrogallol und Hexahydroxybenzol. Erfindungsgemäß bevorzugte Vitis vinifera-Polyphenole sind ausgewählt aus den Mono- und Polyhydroxystilbenen, den Oligomeren von Polyhydroxystilbenen, sowie den Alkylethern und Estern dieser Stilbenverbindungen. Unter Polyhydroxystilbenen werden erfindungsgemäß Stilbene verstanden, die mit 2, 3, 4, 5, 6, 7, 8, 9 oder 10 Hydroxygruppen an den beiden Phenylresten substituiert sind, wobei diese verestert oder verethert sein können. Besonders bevorzugte Hydroxystilbene sind ausgewählt aus Resveratrol (trans-Stilben-3,4'-5-triol), Pinosylvin und Piceatannol. Unter den Oligomeren von Hydroxystilbenen werden erfindungsgemäß Dimere, Trimere, Tetramere, Pentamere und Hexamere von Mono- und Polyhydroxystilbenen verstanden. Besonders bevorzugt sind die Dimere, Trimere und Tetramere, außerordentlich bevorzugt sind die Dimere von Polyhydroxystilbenen. Über den Angriff einer Hydroxylgruppe an die ethylenische Doppelbindung eines weiteren Hydroxystilben-Moleküls unter Ausbildung eines Dihydrofuranrings können Hydroxystilbene oligomerisieren. Bevorzugte Beispiele für die Oligomere von Hydroxystilbenen sind die Dimere des Resveratrols, insbesondere des (E)-Resveratrols, die so genannten Viniferine, insbesondere das trans-epsilon-Viniferin. Bevorzugte Trimere und Tetramere des Resveratrols sind Vitisin E und Vitisin D. Weitere bevorzugte Vitis vinifera-Polyphenole sind Flavone, Anthocyanidine, insbesondere Delphinidin, Maldivin und Pelargonidin, Catechine, Epicatechine, Usninsäure, und als Tannine Gallussäure und die Derivate der Gallussäure, Digallussäure und Digalloylgallussäure. Besonders bevorzugte Polyphenole sind die monomeren Catechine, das heißt die Derivate der Flavan-3-ole, und Leukoanthocyanidine, das heißt die Derivate der Leukoanthocyanidine, die bevorzugt in 5,7,3',4',5'-Stellung phenolische Hydroxygruppen tragen, bevorzugt Epicatechin und Epigallocatechin, sowie die daraus durch Selbstkondensation entstehenden Gerbstoffe. Solche Gerbstoffe werden bevorzugt nicht in isolierter Reinsubstanz, sondern als Extrakte gerbstoffreicher Vitis vinifera-Pflanzenteile eingesetzt, insbesondere der Fruchtschale, den Samen, den Blättern, den Stielen, Zweigen, Ästen und der Rinde. Weitere bevorzugte Vitis vinifera-Polyphenole sind Flavonole, insbesondere Quercetin und Merycetin. Weitere bevorzugte Vitis vinifera-Polyphenole sind Proanthocyanidine, insbesondere Proanthocyanidin B1, Proanthocyanidin B2, Proanthocyanidin B3 und Proanthocyanidin B4. Weitere bevorzugte Vitis vinifera-Polyphenole sind Di-, Tri-, Tetra- und Pentahydroxyzimtsäuren, insbesondere Chlorogensäure und Kaffeesäure.
Die Polyphenol-Mischungen, die aus Vitis vinifera extrahiert sind, haben sich in der erfindungsgemäßen Kombination mit dem Tetrapeptid Lysin-Asparaginsäure-Isoleucin-Citrullin Lys-Asp-Ile-Cit-NH₂ als besonders stark antioxidativ erwiesen.
Erfindungsgemäß bevorzugte Zusammensetzungen enthalten - bezogen auf ihr Gewicht - mindestens ein Vitis vinifera-Polyphenol in einer Gesamtmenge von 0,001 - 2 Gew.-%, bevorzugt 0,005 - 1 Gew.-%, besonders bevorzugt 0,01 - 0,5 Gew.-%, außerordentlich bevorzugt 0,05 - 0,2 Gew.-%.
Weitere erfindungsgemäß bevorzugte Zusammensetzungen enthalten - bezogen auf ihr Gewicht - das Tetrapeptid Lysin-Asparaginsäure-Isoleucin-Citrullin Lys-Asp-Ile-Cit-NH₂ in einer Menge von 0,00001 - 0,001 Gew.-% und mindestens ein Vitis vinifera-Polyphenol in einer Gesamtmenge von 0,001 - 2 Gew.-%, bevorzugt 0,005 - 1 Gew.-%, besonders bevorzugt 0,01 - 0,5 Gew.-%, außerordentlich bevorzugt 0,05 - 0,2 Gew.-%.
In einer weiteren bevorzugten Ausführungsform sind die erfindungsgemäß in ihrer Wirkung gesteigerten Antioxidantien aus Ubichinonen und Ubichinolen ausgewählt.
Ubichinone stellen die am weitesten verbreiteten und damit am besten untersuchten Biochinone dar. Ubichinone werden je nach Zahl der in der Seitenkette verknüpften Isopren-Einheiten als Q-1, Q-2, Q-3 usw. oder nach Anzahl der C-Atome als U-5, U-10, U-15 usw. bezeichnet. Sie treten bevorzugt mit bestimmten Kettenlängen auf, z. B. in einigen Mikroorganismen und Hefen mit n=6. Bei den meisten Säugetieren, einschließlich dem Menschen, überwiegt Q-10. Ubichinone dienen den Organismen als Elektronenüberträger in der Atmungskette. Sie befinden sich in den Mitochondrien, wo sie die cyclische Oxidation und Reduktion der Substrate des CitronensäureCyclus ermöglichen.
Die folgende allgemeine Formel stellt die oxidierte Ubichinon-Form dar. Erfindungsgemäß bevorzugt sind Ubichinone der folgenden Formel mit n = 6, 7, 8, 9 oder 10. Erfindungsgemäß besonders bevorzugt ist das Ubichinon der Formel mit n = 10, auch bekannt als Coenzym Q10.

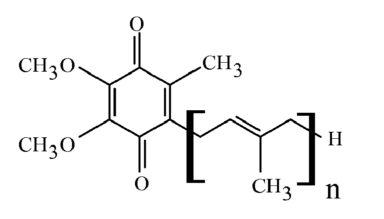

Die reduzierte Form der Ubichinone, die Ubichinole, stellt die antioxidativ wirksame Form dar. Wegen der besseren Lagerfähigkeit wird üblicherweise das Ubichinon, bevorzugt das Ubichinon Q-10, eingesetzt, das auf der Haut zunächst in das Ubichinol, bevorzugt das Ubichinol Q-10, umgewandelt wird. Erfindungsgemäß besonders bevorzugt liegt schon das in der erfindungsgemäßen Zusammensetzung eingesetzte Ubichinon/Ubichinol zu mindestens 10 %, bevorzugt zu mindestens 30 %, besonders bevorzugt zu mindestens 50 %, in der reduzierten Form vor. Die nachfolgenden Mengenangaben beziehen sich auf die Gesamtmenge an Ubichinon und Ubichinol. Erfindungsgemäß bevorzugte Zusammensetzungen enthalten - bezogen auf ihr Gewicht - mindestens ein Ubichinon und/oder Ubichinol in einer Gesamtmenge von 0,001 - 2 Gew.-%, bevorzugt 0,005 - 1 Gew.-%, besonders bevorzugt 0,01 - 0,5 Gew.-%, außerordentlich bevorzugt 0,05 - 0,2 Gew.-%.

Weitere erfindungsgemäß bevorzugte Zusammensetzungen enthalten - bezogen auf ihr Gewicht
- Ubichinon-10 und/oder Ubichinol-10 in einer Gesamtmenge von 0,001 - 2 Gew.-%, bevorzugt 0,005 - 1 Gew.-%, besonders bevorzugt 0,01 - 0,5 Gew.-%, außerordentlich bevorzugt 0,05 - 0,2 Gew.-%.

Weitere erfindungsgemäß bevorzugte Zusammensetzungen enthalten - bezogen auf ihr Gewicht
- das Tetrapeptid Lysin-Asparaginsäure-Isoleucin-Citrullin Lys-Asp-Ile-Cit-NH₂ in einer Menge von 0,00001 - 0,001 Gew.-% und mindestens ein Ubichinon und/oder Ubichinol in einer Gesamtmenge von 0,001 - 2 Gew.-%, bevorzugt 0,005 - 1 Gew.-%, besonders bevorzugt 0,01 - 0,5 Gew.-%, außerordentlich bevorzugt 0,05 - 0,2 Gew.-%.

Weitere erfindungsgemäß bevorzugte Zusammensetzungen enthalten - bezogen auf ihr Gewicht
- das Tetrapeptid Lysin-Asparaginsäure-Isoleucin-Citrullin Lys-Asp-Ile-Cit-NH₂ in einer Menge von 0,00001 - 0,001 Gew.-% und Ubichinon-10 und/oder Ubichinol-10 in einer Gesamtmenge von 0,001 - 2 Gew.-%, bevorzugt 0,005 - 1 Gew.-%, besonders bevorzugt 0,01 - 0,5 Gew.-%, außerordentlich bevorzugt 0,05 - 0,2 Gew.-%.

In einer weiteren bevorzugten Ausführungsform sind die erfindungsgemäß in ihrer Wirkung gesteigerten Antioxidantien aus mindestens einer Superoxiddismutase (Superoxid-Oxidoreduktase, EC 1.15.1.1) ausgewählt.

Der Begriff "Superoxiddismutase" ist eine Sammelbezeichnung für in allen Aerobiern vorkommende Metalloproteine, die als Oxidoreduktasen das Radikal-Ion Hyperoxid (Superoxid) in Wasserstoffperoxid und Sauerstoff umwandeln: 20₂ ^{■-} + 2H⁺ → H₂O₂ + O₂. Die Superoxiddismutasen scheinen im Organismus für die Entgiftung toxischer Sauerstoff-Spezies verantwortlich zu sein, insbesondere des Hyperoxids, das im Körper aus molekularem Sauerstoff entsteht, z. B. durch die NADPH-Oxidase der phagocytierenden Makrophagen und neutrophilen Granulocyten bei Entzündungen (respiratory burst), durch ionisierende Strahlung usw. Erfindungsgemäß besonders bevorzugt sind Kupfer/ Zink-Superoxiddismutasen. Das nicht blaue Kupferprotein mit Mᵣ im Bereich von 31300 bis 32000 Dalton enthält in seinen beiden identischen Untereinheiten ein Kupfer- und ein Zink-Atom und ist sehr stabil gegen Denaturierung. Verschiedene solcher Cu₂Zn₂-Superoxiddismutasen bzw. Kupfer/Zink-Superoxiddismutasen findet man in fast allen Geweben von Eukaryonten.

Erfindungsgemäß bevorzugte Zusammensetzungen enthalten - bezogen auf ihr Gewicht - mindestens eine Superoxiddismutase in einer Gesamtmenge von 0,001 - 1 Gew.-%, bevorzugt 0,005 - 0,1 Gew.-%, besonders bevorzugt 0,01 - 0,05 Gew.-%, außerordentlich bevorzugt 0,03 - 0,04 Gew.-%.

Weitere erfindungsgemäß bevorzugte Zusammensetzungen enthalten - bezogen auf ihr Gewicht
- mindestens eine Kupfer/Zink-Superoxiddismutase in einer Gesamtmenge von 0,001 - 1 Gew.-%, bevorzugt 0,005 - 0,1 Gew.-%, besonders bevorzugt 0,01 - 0,05 Gew.-%, außerordentlich bevorzugt 0,03 - 0,04 Gew.-%.

Weitere erfindungsgemäß bevorzugte Zusammensetzungen enthalten - bezogen auf ihr Gewicht
- das Tetrapeptid Lysin-Asparaginsäure-Isoleucin-Citrullin Lys-Asp-Ile-Cit-NH₂ in einer Menge von 0,00001 - 0,001 Gew.-% und mindestens eine Superoxiddismutase in einer Gesamtmenge von 0,001 - 1 Gew.-%, bevorzugt 0,005 - 0,1 Gew.-%, besonders bevorzugt 0,01 - 0,05 Gew.-%, außerordentlich bevorzugt 0,03 - 0,04 Gew.-%.

Weitere erfindungsgemäß bevorzugte Zusammensetzungen enthalten - bezogen auf ihr Gewicht
- das Tetrapeptid Lysin-Asparaginsäure-Isoleucin-Citrullin Lys-Asp-Ile-Cit-NH₂ in einer Menge von 0,00001 - 0,001 Gew.-% und mindestens eine Kupfer/Zink-Superoxiddismutase in einer Gesamtmenge von 0,001 - 1 Gew.-%, bevorzugt 0,005 - 0,1 Gew.-%, besonders bevorzugt 0,01
- 0,05 Gew.-%, außerordentlich bevorzugt 0,03 - 0,04 Gew.-%.

In einer weiteren bevorzugten Ausführungsform sind die erfindungsgemäß in ihrer Wirkung gesteigerten Antioxidantien aus mindestens einer Verbindung aus der Gruppe der Ellagsäure und/oder Ellagate ausgewählt. Ellagsäure (2,3,7,8-Tetrahydroxy[1]benzopyrano-[5,4,3-*cde*][1]benzopyran-5,10-dion, siehe unten stehende Formel) 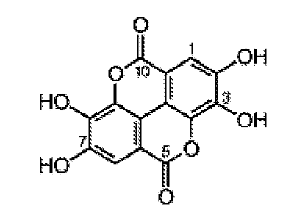 ist frei und in Form von Glycosiden und/oder Methylethern im Pflanzenreich weit verbreitet, besonders in Galläpfeln, Blattgallen, auch in den Blättern von *Eucalyptus*-Arten, in Eichen und Euphorbien. Neben der Ellagsäure sind insbesondere die physiologisch verträglichen Salze der Ellagsäure, die Ellagate, bevorzugt, insbesondere ausgewählt aus den Ammonium-, Alkylammonium-, Monoalkylammonium-, Dialkylammonium-, Trialkylammonium-, Alkalimetall-, Magnesium-, Calcium-, Aluminium-, Zink- und Mangan-Salzen. Bevorzugt sind die Natrium-, Kalium-, Magnesium-, Aluminium-, Zink- und Mangan-Salze. Außerordentlich bevorzugt ist Natriumellagat.

Bevorzugte erfindungsgemäße Zusammensetzungen sind **dadurch gekennzeichnet, dass** sie - bezogen auf ihr Gewicht - Ellagsäure und/oder Ellagate in einer Gesamtmenge von 0,001 - 2 Gew.-%, bevorzugt 0,005 - 1 Gew.-%, besonders bevorzugt 0,01 - 0,5 Gew.-%, außerordentlich bevorzugt 0,05 - 0,2 Gew.-%, enthalten.

Weitere erfindungsgemäß bevorzugte Zusammensetzungen enthalten - bezogen auf ihr Gewicht - das Tetrapeptid Lysin-Asparaginsäure-Isoleucin-Citrullin Lys-Asp-Ile-Cit-NH₂ in einer Menge von 0,00001 - 0,001 Gew.-% und Ellagsäure und/oder Ellagate in einer Gesamtmenge von 0,001 - 2 Gew.-%, bevorzugt 0,005 - 1 Gew.-%, besonders bevorzugt 0,01 - 0,5 Gew.-%, außerordentlich bevorzugt 0,05 - 0,2 Gew.-%.

Weitere erfindungsgemäß bevorzugte kosmetische Zusammensetzungen sind **dadurch gekennzeichnet, dass** das Tetrapeptid Lysin-Asparaginsäure-Isoleucin-Citrullin Lys-Asp-Ile-Cit-NH₂ am Lysin-Rest mit einem C₂ - C₂₄-Acylrest N-acyliert ist.

Bevorzugte C₂ - C₂₄-Acylreste, mit denen das Tetrapeptid Lysin-Asparaginsäure-Isoleucin-Citrullin Lys-Asp-Ile-Cit-NH₂ am Lysin-Rest acyliert sein kann, sind ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten. Als besonders bevorzugt hat sich der Palmitoyl-Rest herausgestellt.

Erfindungsgemäß besonders bevorzugte kosmetische Zusammensetzungen sind **dadurch gekennzeichnet, dass** das Tetrapeptid Lysin-Asparaginsäure-Isoleucin-Citrullin Lys-Asp-Ile-Cit-NH₂ am Lysin-Rest mit einem Palmitoyl-Rest N-acyliert ist.

Weitere erfindungsgemäß bevorzugte kosmetische Zusammensetzungen sind **dadurch gekennzeichnet, dass** mindestens eine basische Aminosäure, ausgewählt aus Lysin, Arginin und Histidin, sowie Mischungen dieser basischen Aminosäuren, enthalten ist.

Es hat sich überraschend gezeigt, dass die Steigerung der antioxidativen Wirkung der erfindungsgemäß beanspruchten Antioxidantien durch das Tetrapeptid Lysin-Asparaginsäure-Isoleucin-Citrullin Lys-Asp-Ile-Cit-NH₂ und/oder die Verfügbarkeit des Tetrapeptids Lysin-Asparaginsäure-Isoleucin-Citrullin positiv beeinflusst werden kann durch den Zusatz von mindestens einer basischen Aminosäure, ausgewählt aus Lysin, Arginin und Histidin, sowie Mischungen dieser basischen Aminosäuren. Besonders bevorzugt ist hierbei das Lysin, insbesondere L-Lysin. Besonders bevorzugte erfindungsgemäße Zusammensetzungen enthalten mindestens eine basische Aminosäure, ausgewählt aus Lysin, Arginin und Histidin, sowie Mischungen dieser basischen Aminosäuren, in einer Gesamtmenge von 0,01 - 1 Gew.-%, bevorzugt 0,03 - 0,5 Gew.-%, besonders bevorzugt 0,05 - 0,2 Gew.-%, außerordentlich bevorzugt 0,06 - 0,1 Gew.-%, jeweils bezogen auf das Gesamtgewicht der erfindungsgemäßen Zusammensetzung.

Weitere erfindungsgemäß bevorzugte Zusammensetzungen enthalten - bezogen auf ihr Gewicht
- das Tetrapeptid Lysin-Asparaginsäure-Isoleucin-Citrullin Lys-Asp-Ile-Cit-NH₂ in einer Menge von 0,00001 - 0,001 Gew.-% und mindestens eine basische Aminosäure, ausgewählt aus Lysin, Arginin und Histidin, sowie Mischungen dieser basischen Aminosäuren, in einer Gesamtmenge von 0,01 - 1 Gew.-%, bevorzugt 0,03 - 0,5 Gew.-%, besonders bevorzugt 0,05 - 0,2 Gew.-%, außerordentlich bevorzugt 0,06 - 0,1 Gew.-%.

Weitere erfindungsgemäß bevorzugte Zusammensetzungen enthalten - bezogen auf ihr Gewicht
- das Tetrapeptid Lysin-Asparaginsäure-Isoleucin-Citrullin Lys-Asp-Ile-Cit-NH₂ in einer Menge von 0,00001 - 0,001 Gew.-% und Lysin in einer Gesamtmenge von 0,01 - 1 Gew.-%, bevorzugt 0,03 - 0,5 Gew.-%, besonders bevorzugt 0,05 - 0,2 Gew.-%, außerordentlich bevorzugt 0,06 - 0,1 Gew.-%.

Weitere erfindungsgemäß bevorzugte kosmetische Zusammensetzungen sind **dadurch gekennzeichnet, dass** das Tetrapeptid Lysin-Asparaginsäure-Isoleucin-Citrullin Lys-Asp-Ile-Cit-NH₂ und die Gesamtmenge an mindestens einer basischen Aminosäure, die ausgewählt ist aus Lysin, Arginin und Histidin, in einem Gewichtsverhältnis von 1:100 bis 1:1000, bevorzugt 1:200 - 1:700, besonders bevorzugt 1:300 - 1:600, außerordentlich bevorzugt 1:400 - 1:500, enthalten sind. Entsprechende Gewichtsverhältnisse haben sich für den erfindungsgemäßen Zweck als besonders vorteilhaft erwiesen.

Weiterhin hat es sich für die Optimierung der antioxidativen und damit auch Antifalten-Wirkung als besonders vorteilhaft erwiesen, wenn das Tetrapeptid Lysin-Asparaginsäure-Isoleucin-Citrullin Lys-Asp-Ile-Cit-NH₂ in verkapselter Form, insbesondere in Vesikeln oder Liposomen verkapselt, vorliegt.

Unter dem Begriff "in verkapselter Form" werden Aggregate verstanden, die mindestens einen festen oder flüssigen Kern aufweisen, der das Tetrapeptid Lysin-Asparaginsäure-Isoleucin-Citrullin Lys-Asp-Ile-Cit-NH₂ enthält, der von mindestens einer kontinuierlichen Hülle umschlossen ist. Besonders bevorzugt handelt es sich um mit filmbildenden Polymeren umhüllte feindisperse flüssige oder feste Benefit-haltige Phasen, bei deren Herstellung sich die Polymere nach Emulgierung und Koazervation oder Grenzflächenpolymerisation auf dem einzuhüllenden Material niederschlagen. Die Kapseln lassen sich wie Pulver trocknen. Neben einkernigen Kapseln sind auch mehrkernige Aggregate bekannt, die zwei oder mehr Kerne im kontinuierlichen Hüllmaterial verteilt enthalten. Ein- oder mehrkernige Kapseln können zudem von einer zusätzlichen zweiten, dritten etc. Hülle umschlossen sein. Bevorzugt sind einkernige Kapseln mit einer kontinuierlichen Hülle. Die Hülle kann aus natürlichen, halbsynthetischen oder synthetischen Materialien bestehen. Natürliche Hüllmaterialien sind beispielsweise Gummi arabicum, Agar Agar, Agarose, Maltodextrine, Alginsäure bzw. ihre Salze, z. B. Natrium- oder Calciumalginate, Fette und Fettsäuren, Cetylalkohol, Collagen, Chitosan, Phospholipide, insbesondere Lecithine, die gehärtet (hydriert) oder ungehärtet (nicht hydriert) sein können, Gelatine, Albumin, Schelllack, Polysaccharide, wie Stärke oder Dextran, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohole oder Polyvinylpyrrolidon. Besonders bevorzugte erfindungsgemäße Produkte sind **dadurch gekennzeichnet, dass** die Benefit-Zusammensetzung Kapseln enthält, deren Hüllsubstanz ausgewählt ist aus der Gruppe Gummi arabicum, Agar, Agarose, Maltodextrine, Alginsäure, Alginate, Fette, Fettsäuren, Cetylalkohol, Collagen, Chitosan, Phospholipide, insbesondere Lecithine, die gehärtet (hydriert) oder ungehärtet (nicht hydriert) sein können, Gelatine, Albumin, Schellack, Polysaccharide, Cellulosen, Celluloseester, Celluloseether, Stärkeether, Stärkeester, Polyacrylate, Polyamide, Polyvinylalkohole und Polyvinylpyrrolidon.

Die Tetrapeptid-haltigen Kapseln können im herstellungsbedingten Rahmen eine beliebige Form aufweisen, sie sind jedoch bevorzugt näherungsweise kugelförmig. Ihr Durchmesser entlang ihrer größten räumlichen Ausdehnung kann je nach den in ihrem Inneren enthaltenen Substanzen und der Anwendung zwischen 10 nm (visuell nicht als Kapsel erkennbar) und 10 mm liegen. Bevorzugt sind Kapseln mit einem Durchmesser von 20 - 650 nm, vorzugsweise 60 - 250 nm.

Die Kapseln sind nach im Stand der Technik bekannten Verfahren zugänglich, wobei der Koazervation und der Grenzflächenpolymerisation die größte Bedeutung zukommt. Als Kapseln lassen sich sämtliche auf dem Markt angebotenen tensidstabilen Kapseln einsetzen, beispielsweise die Handelsprodukte (in Klammern angegeben ist jeweils das Hüllmaterial) Hallcrest Microcapsules (Gelatine, Gummi Arabicum), Coletica Thalaspheres (maritimes Collagen), Lipotec Millicapseln (Alginsäure, Agar-Agar), Induchem Unispheres (Lactose, mikrokristalline Cellulose, Hydroxypropylmethylcellulose), Unicerin C30 (Lactose, mikrokristalline Cellulose, Hydroxypropylmethylcellulose), Kobo Glycospheres (modifizierte Stärke, Fettsäureester, Phospholipide), Softspheres (modifiziertes Agar-Agar) und Kuhs Probiol Nanospheres (Phospholipide).

Die Freisetzung des Tetrapeptids aus den Kapseln erfolgt üblicherweise während der Anwendung der sie enthaltenden Zubereitungen durch Zerstörung der Hülle infolge mechanischer, thermischer, chemischer oder enzymatischer Einwirkung.

Besonders bevorzugt liegt das Tetrapeptid Lysin-Asparaginsäure-Isoleucin-Citrullin Lys-Asp-Ile-Cit-NH₂ verkapselt in Phospholipid-Vesikeln oder Phospholipid-Liposomen, insbesondere in Lecithin-Vesikeln oder Lecithin-Liposomen, vor.

Weiterhin hat sich als erfindungsgemäß besonders vorteilhaft herausgestellt, wenn die erfindungsgemäßen kosmetischen Zusammensetzungen in Form einer Öl-in-Wasser-Emulsion vorliegen. Dies hat sich als besonders vorteilhaft für die erfindungsgemäße Steigerung des antioxidativen Potenzials der Carotinoide, insbesondere von Astaxanthin, Lutein und Zeaxanthin, sowie der Vitis vinifera-Polyphenole erwiesen.

Die erfindungsgemäßen Zusammensetzungen können weitere optionale Inhaltsstoffe enthalten. Besonders bevorzugt ist ein Gehalt an mindestens einer Feuchtigkeit spendenden Substanz. Diese können einen Beitrag zur Erhaltung oder Wiederherstellung der Hautfeuchtigkeit leisten und dem Austrocknen der Haut entgegenwirken. Hierzu eignen sich vor allem wasserlösliche mehrwertige C₂ - C₉-Alkanole mit 2 - 6 Hydroxylgruppen und/oder wasserlösliche Polyethylenglycole mit 3 - 20 Ethylenoxid-Einheiten sowie Mischungen hiervon. Bevorzugt sind diese Komponenten ausgewählt aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit sowie Mischungen der vorgenannten Substanzen. Geeignete wasserlösliche Polyethylenglycole sind ausgewählt aus PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 bevorzugt sind. Auch Zucker und bestimmte Zuckerderivate wie Fucose, Rhamnose, Fructose, Glucose, Maltose, Maltitol, Mannit, Inosit, Sucrose, Trehalose und Xylose sind erfindungsgemäß geeignet. Bevorzugte erfindungsgemäße Hautbehandlungsmittel sind **dadurch gekennzeichnet, dass** das mindestens eine wasserlösliche mehrwertige C₂ - C₉-Alkanol mit 2 - 6 Hydroxylgruppen und/oder mindestens eine wasserlösliche Polyethylenglycol mit 3 - 20 Ethylenoxid-Einheiten ausgewählt ist aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit sowie Mischungen der vorgenannten Substanzen. Besonders bevorzugte erfindungsgemäße Hautbehandlungsmittel sind **dadurch gekennzeichnet, dass** das mindestens eine wasserlösliche mehrwertige C₂ - C₉-Alkanol mit 2 - 6 Hydroxylgruppen und/oder mindestens eine wasserlösliche Polyethylenglycol mit 3 - 20 Ethylenoxid-Einheiten insgesamt in Mengen von 3 - 30 Gew.-%, bevorzugt 8 - 25 Gew.-%, besonders bevorzugt 10 - 18 Gew.-%, jeweils bezogen auf die Gesamtzusammensetzung, enthalten ist.

Weitere bevorzugte Feuchtigkeit spendende Substanzen sind ausgewählt aus Harnstoff und alkyl-oder hydroxyalkylsubstituierten Harnstoffverbindungen sowie - besonders bevorzugt - Mischungen hiervon.

Erfindungsgemäß bevorzugte alkyl- oder hydroxyalkylsubstituierte Harnstoffverbindungen weisen die allgemeine Formel (UREA-1) auf, 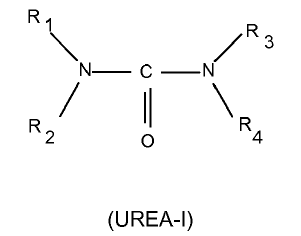 in der R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-Hydroxyalkyl-Gruppe, die mit 1 bis 5 Hydroxylgruppen oder C₁-C₄-Hydroxyalkyl-Gruppen substituiert ist, stehen, mit der Maßgabe, dass mindestens einer der Reste R₁ - R₄ eine C₂-C₆-Hydroxyalkyl-Gruppe, die mit 1 bis 5 Hydroxylgruppen oder C₁-C₄-Hydroxyalkyl-Gruppen substituiert ist, darstellt. Erfindungsgemäß besonders bevorzugte alkyl- oder hydroxyalkylsubstituierte Harnstoffverbindungen der allgemeinen Formel (UREA-1) sind ausgewählt aus Verbindungen, bei denen jeweils mindestens einer der Reste R₁ und R₂ bzw. R₃ und R₄ eine C₂-C₆-Hydroxyalkyl-Gruppe, die mit 1 bis 5 Hydroxylgruppen oder C₁-C₄-Hydroxyalkyl-Gruppen substituiert ist, darstellt. Besonders bevorzugte C₂-C₆-Hydroxyalkyl-Gruppen, die mit 1 bis 5 Hydroxylgruppen oder C₁-C₄-Hydroxyalkyl-Gruppen substituiert sind, sind ausgewählt aus 2-Hydroxyethyl-, 3-Hydroxypropyl-, 2-Hydroxypropyl, 2-Hydroxyisopropyl- und 4-Hydroxybutyl-Gruppen, wobei die 2-Hydroxyethyl-Gruppe außerordentlich bevorzugt ist. Ebenfalls außerordentlich bevorzugt ist Bis-N,N'-(2-Hydroxyethyl)-harnstoff. Harnstoff selbst ist ebenfalls besonders bevorzugt. Weiterhin bevorzugt ist es, Mischungen von Harnstoff und alkyl- oder hydroxyalkylsubstituierten Harnstoffverbindungen einzusetzen. Außerordentlich bevorzugt sind Mischungen von Harnstoff und Bis-N,N'-(2-Hydroxyethyl)-harnstoff. Erfindungsgemäß besonders bevorzugte Zusammensetzungen sind **dadurch gekennzeichnet, dass** sie mindestens einen alkyl- oder hydroxyalkylsubstituierten Harnstoff gemäß Formel (UREA-1), insbesondere N,N'-Bis-(2-hydroxyethyl)harnstoff, in einer Gesamtmenge von 0,01 - 50 Gew.-%, bevorzugt 0,1 - 20 Gew.-%, besonders bevorzugt 1 - 10 Gew.-% und außerordentlich bevorzugt 2 - 5 Gew.-%, bezogen auf die gesamte Zusammensetzung, enthalten. N,N'-Bis-(2-hydroxyethyl)-harnstoff, bei Raumtemperatur ein kristalliner Feststoff, ist beispielsweise als Handelsprodukt Hydrovance von Akzo Nobel als ca. 45 - 55 %ige wässrige Lösung mit einem Gehalt an Harnstoff und Ammoniumlactat erhältlich.

Auch filmbildende Substanzen sind bevorzugte optionale Zusatzstoffe. Filmbildner bilden einen schützenden, stabilisierenden Film auf Oberflächen, vorzugsweise Haut, Haar oder Nägeln. Mit Bezug auf die erfindungsgemäßen Mittel wirkt dieser Film vorzugsweise feuchtigkeitsspeichernd. Bevorzugte feuchtigkeitsspeichernde Filmbildner sind ausgewählt aus Polysacchariden, die mindestens einen Desoxyzucker-Baustein enthalten, besonders bevorzugt aus Polysacchariden mit der INCI-Bezeichnung Biosaccharide Gum-1, erhältlich beispielsweise als Handelsprodukt Fucogel^{®} von Solabia, Polysacchariden mit der INCI-Bezeichnung Biosaccharide Gum-2, erhältlich beispielsweise als Handelsprodukt Rhamnosoft^{®} von Solabia, Polysacchariden mit der INCI-Bezeichnung Biosaccharide Gum-3, erhältlich beispielsweise als Handelsprodukt Fucogenol^{®} von Solabia, und Polysacchariden mit der INCI-Bezeichnung Biosaccharide Gum-4, erhältlich beispielsweise als Handelsprodukt Glycofilm^{®} von Solabia, weiterhin Mischungen der vorgenannten, mindestens einen Desoxyzucker-Baustein enthaltenden Polysaccharide, beispielsweise der Mischung aus Biosaccharide Gum-2 und Biosaccharide Gum-3, erhältlich als Handelsprodukt Elastinol plus^{®} von Solabia, Glycosaminoglycanen, besonders bevorzugt Hyaluronsäure, Dextran, Dextransulfat, Chondroitin-4-sulfat und Chondroitin-6-sulfat.

In einer bevorzugten Ausführungsform enthalten die erfindungsgemäßen Zusammensetzungen zusätzlich zu dem Tetrapeptid Lysin-Asparaginsäure-Isoleucin-Citrullin Lys-Asp-Ile-Cit-NH₂ mindestens einen hiervon verschiedenen kosmetischen Wirkstoff, der ausgewählt ist aus Monomeren, Oligomeren und Polymeren von Aminosäuren, N-C₂-C₂₄-Acylaminosäuren und/oder den Estern und/oder den physiologisch verträglichen Salzen dieser Substanzen, sowie Mischungen dieser Wirkstoffe.

Die Monomere der Aminosäuren und/oder der N-C₂-C₂₄-Acylaminosäuren sind bevorzugt ausgewählt aus Alanin, Arginin, Asparagin, Asparaginsäure, Canavanin, Citrullin, Cystein, Cystin, Desmosin, Dipalmitoylhydroxyprolin, Glutamin, Glutaminsäure, Glycin, Histidin, Homophenylalanin, Hydroxylysin, Hydroxyprolin, Isodesmosin, Isoleucin, Leucin, Lysin, Methionin, Methylnorleucin, Ornithin, Phenylalanin, Prolin, Pyroglutaminsäure, Sarcosin, Serin, Taurin, Threonin, Thyroxin, Tryptophan, Tyrosin, Valin, N-Acetyl-L-cystein, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Besonders bevorzugt sind Lysin, Serin, N-Acetyl-L-cystein, Zink- und Natriumpyroglutamat und Natriumlauroylglutamat.

Der C₂ - C₂₄-Acylrest, mit dem die genannten Aminosäuren an der Aminogruppe derivatisiert sein können, ist bevorzugt ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten.

Mit den vorgenannten C₂ - C₂₄-Acylresten können die Aminosäuren, die eine OH-Gruppe tragen, auch an dieser OH-Gruppe verestert sein. Ein erfindungsgemäß bevorzugtes Beispiel hierfür ist Hydroxyprolin, das mit zwei, bevorzugt linearen, C₂-C₂₂-Fettsäureresten N-acyliert und verestert ist, besonders bevorzugt Dipalmitoylhydroxyprolin, das z. B. unter der Bezeichnung Sepilift DPHP von der Firma Seppic erhältlich ist.

Die physiologisch verträglichen Salze der erfindungsgemäß bevorzugten Wirkstoffe, die Säuregruppen enthalten und Salze bilden können, sind ausgewählt aus den Ammonium-, Alkalimetall-, Magnesium-, Calcium-, Aluminium-, Zink- und Mangan-Salzen. Bevorzugt sind die Natrium-, Kalium-, Magnesium-, Aluminium-, Zink- und Mangan-Salze.

Unter Aminosäureoligomeren werden erfindungsgemäß Peptide mit 2 - 30, bevorzugt 2 - 15, Aminosäuren, verstanden. Die Oligomere der Aminosäuren und/oder der N-C₂-C₂₄-Acylaminosäuren sind bevorzugt ausgewählt aus Di-, Tri-, Tetra-, Penta-, Hexa- oder Pentadecapeptiden, die N-acyliert und/oder verestert sein können. Zahlreiche dieser Aminosäureoligomere stimulieren die Collagensynthese beziehungsweise sind in der Lage, Zellen des Immunsystems, wie Mastzellen und Makrophagen, zu rekrutieren, die dann über die Freisetzung von Wachstumsfaktoren Reparaturprozesse im Gewebe, z.B. die Collagensynthese, induzieren, beziehungsweise sind in der Lage, an die Sequenz Arg-Phe-Lys in Thrombospondin I (TSP-1) zu binden und damit aktives TGF-β (*tissue growth factor*), der die Synthese von Collagen in dermalen Fibroblasten induziert, freizusetzen. Derartige Aminosäureoligomere können als Wirkstoffe gegen die Hautalterung verwendet werden.

Erfindungsgemäß bevorzugte, gegebenenfalls N-acylierte und/oder veresterte Dipeptide sind Acetyl-Citrullyl-Arginin (z. B. Exsy-Algine von Exsymol mit der INCI-Bezeichnung Acetyl Citrull Amido Arginine), Tyr-Arg (Dipeptide-1), Val-Trp (Dipeptide-2), Asn-Phe, Asp-Phe, N-Palmitoyl-β-Ala-His, N-Acetyl-Tyr-Arg-hexyldecylester (z. B. Calmosensine von Sederma), Carnosin (β-Ala-His) und N-Palmitoyl-Pro-Arg. Erfindungsgemäß bevorzugte, gegebenenfalls N-acylierte und/oder veresterte Tripeptide sind Gly-His-Lys, das z. B. unter der Bezeichnung "Omega-CH-Aktivator" von der Firma GfN oder in acylierter Form (N-Palmitoyl-Gly-His-Lys) unter der Bezeichnung Biopeptide CL von Sederma erhältlich ist, aber (in acylierter Form) auch einen Bestandteil des Produktes Matrixyl 3000 von Sederma darstellt. Das Tripeptid Gly-His-Lys kann auch als Kupfersalz (Cu²⁺) eingesetzt werden und ist als solches über ProCyte Corporation zu beziehen. Weiterhin können Analoga von Gly-His-Lys eingesetzt werden, wobei maximal zwei Aminosäuren durch geeignete andere Aminosäuren substituiert sind. Zur Substitution von Gly sind erfindungsgemäß Ala, Leu und Ile geeignet. Die erfindungsgemäß bevorzugten Aminosäuren, die His oder Lys ersetzen können, beinhalten eine Seitenkette mit einem Stickstoffatom, das bei pH 6 überwiegend geladen vorliegt, z. B. Pro, Lys, Arg, His, Desmosin und Isodesmosin. Besonders bevorzugt wird Lys durch Arg, Orn, oder Citrullin ersetzt. Ein weiteres erfindungsgemäß bevorzugtes Tripeptid ist Gly-His-Arg (INCI-Bezeichnung: Tripeptide-3) sowie dessen Derivat N-Myristoyl-Gly-His-Arg, das z. B. unter der Bezeichnung Collasyn 314-GR von Therapeutic Peptide Inc. erhältlich ist; weitere erfindungsgemäß bevorzugte Tripeptide sind ausgewählt aus Lys-Val-Lys, Lys-Val-Dab (Dab = Diaminobuttersäure), Lys-Phe-Lys, Lys-Ile-Lys, Dab-Val-Lys, Lys-Val-Orn, Lys-Val-Dap (Dap = Diaminopropionsäure), Dap-Val-Lys, Palmitoyl-Lys-Val-Lys, z. B. erhältlich von der Firma Pentapharm unter der Bezeichnung SYN^{®}-COLL, Lys-Pro-Val, Tyr-Tyr-Val, Tyr-Val-Tyr, Val-Tyr-Val (Tripeptide-2), Tripeptide-4 (z. B. ATPeptide), His-Ala-Orn N-Elaidoyl-Lys-Phe-Lys und N-Acetyl-Arg-Lys-Arg-NH₂.

Erfindungsgemäß bevorzugte, gegebenenfalls N-acylierte und/oder veresterte Tetrapeptide sind ausgewählt aus Rigin und Rigin-basierten Tetrapeptiden sowie ALAMCAT-Tetrapeptiden. Rigin weist die Sequenz Gly-Gln-Pro-Arg auf. Rigin-basierte Tetrapeptide umfassen die Rigin-Analoga und Rigin-Derivate, insbesondere das erfindungsgemäß besonders bevorzugte N-Palmitoyl-Gly-Gln-Pro-Arg, das z. B. unter der Bezeichnung Eyeliss von Sederma erhältlich ist, aber auch einen Bestandteil des Produktes Matrixyl 3000 von Sederma darstellt. Zu den Rigin-Analoga zählen solche, bei denen die vier Aminosäuren umarrangiert sind und/oder bei denen gegenüber Rigin maximal zwei Aminosäuren substituiert sind, z. B. die Sequenz Ala-Gln-Thr-Arg. Bevorzugt hat mindestens eine der Aminosäuren der Sequenz ein Pro oder Arg und besonders bevorzugt beinhaltet das Tetrapeptid sowohl Pro als auch Arg, wobei ihre Reihenfolge und Position variieren können. Die substituierenden Aminosäuren können aus jeder Aminosäure, die im Folgenden definiert ist, ausgewählt werden. Besonders bevorzugte Rigin-basierte Tetrapetide umfassen: Xaa-Xbb-Arg-Xcc, Xaa-Xbb-Xcc-Pro, Xaa-Xbb-Pro-Arg, Xaa-Xbb-Pro-Xcc, Xaa-Xbb-Xcc-Arg, wobei Xaa, Xbb und Xcc gleiche oder voneinander verschiedene Aminosäuren sein können und wobei Xaa ausgewählt ist aus Gly und den Aminosäuren, die Gly substituieren können, Xbb ausgewählt ist aus Gln und den Aminosäuren, die Gln substituieren können, Xcc ausgewählt ist aus Pro oder Arg und den Aminosäuren, die Pro und Arg substituieren können.

Die bevorzugten Aminosäuren, die Gly ersetzen können, beinhalten eine aliphatische Seitenkette, z. B. β-Ala, Ala, Val, Leu, Pro, Sarcosin (Sar) und Isoleucin (Ile).

Die bevorzugten Aminosäuren, die Gln ersetzen können, beinhalten eine Seitenkette mit einer Aminogruppe, die bei neutralem pH (pH 6-7) überwiegend ungeladen vorliegt, z.B. Asn, Lys, Orn, 5-Hydroxyprolin, Citrullin und Canavanin.

Die bevorzugten Aminosäuren, die Arg ersetzen können, beinhalten eine Seitenkette mit einem Stickstoffatom, das bei pH 6 überwiegend geladen vorliegt, z.B. Pro, Lys, His, Desmosin und Isodesmosin.

Als Rigin-Analoga sind erfindungsgemäß Gly-Gln-Arg-Pro und Val-Val-Arg-Pro bevorzugt.

ALAMCAT-Tetrapeptide sind Tetrapeptide, die mindestens eine Aminosäure mit einer aliphatischen Seitenkette enthalten, z. B. β-Ala, Ala, Val, Leu, Pro, Sarcosin (Sar) und Isoleucin (Ile). Weiterhin beinhalten ALAMCAT-Tetrapeptide mindestens eine Aminosäure mit einer Seitenkette mit einer Aminogruppe, die bei neutralem pH (pH 6-7) überwiegend ungeladen vorliegt, z.B. Gln, Asn, Lys, Orn, 5-Hydroxyprolin, Citrullin und Canavanin. Weiterhin beinhalten ALAMCAT-Tetrapeptide mindestens eine Aminosäure mit einer Seitenkette mit einem Stickstoffatom, das bei pH 6 überwiegend geladen vorliegt, z. B. Arg, Pro, Lys, His, Desmosin und Isodesmosin. Als vierte Aminosäure können ALAMCAT-Tetrapeptide jede beliebige Aminosäure enthalten; bevorzugt ist jedoch auch die vierte Aminosäure aus den drei vorstehend genannten Gruppen ausgewählt. Erfindungsgemäß bevorzugte, gegebenenfalls N-acylierte und/oder veresterte Pentapeptide sind ausgewählt aus Lys-Thr-Thr-Lys-Ser und seinen N-acylierten Derivaten, besonders bevorzugt N-Palmitoyl-Lys-Thr-Thr-Lys-Ser, das unter der Bezeichnung Matrixyl von der Firma Sederma erhältlich ist, weiterhin N-Palmitoyl-Tyr-Gly-Gly-Phe-Met, Val-Val-Arg-Pro-Pro, N-Palmitoyl-Tyr-Gly-Gly-Phe-Leu, Gly-Pro-Phe-Pro-Leu und N-Benzyloxycarbonyl-Gly-Pro-Phe-Pro-Leu (die beiden letztgenannten stellen Serinproteinase-Inhibitoren zur Inhibition der Desquamation dar). Erfindungsgemäß bevorzugte, gegebenenfalls N-acylierte und/oder veresterte Hexapeptide sind Val-Gly-Val-Ala-Pro-Gly und seine N-acylierten Derivate, besonders bevorzugt N-Palmitoyl-Val-Gly-Val-Ala-Pro-Gly, das unter der Bezeichnung Biopeptide EL von der Firma Sederma erhältlich ist, weiterhin Acetyl-Hexapeptide-3 (Argireline von Lipotec), Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience) und Hexapeptide-10 (z. B. Collaxyl von Vincience oder Seriseline von Lipotec), Ala-Arg-His-Leu-Phe-Trp (Hexapeptide-1), Acetyl Hexapeptide-1 (z. B. Modulene von Vincience), Acetyl Glutamyl Hexapeptide-1 (z. B. SNAP-7 von Centerchem), Hexapeptide-2 (z. B. Melanostatine-DM von Vincience), Ala-Asp-Leu-Lys-Pro-Thr (Hexapeptide-3, z. B. Peptide 02 von Vincience), Val-Val-Arg-Pro-Pro-Pro, Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Ala-Arg-His-Methylnorleucin-Homophenylalanin-Trp (Hexapeptide-7), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience), Hexapeptide-10 (z. B. Collaxyl von Vincience oder Seriseline von Lipotec) und Hexapeptide-11 (z. B. Peptamide-6 von Arch Personal Care). Ein erfindungsgemäß bevorzugtes Pentadecapeptid ist z. B. der Rohstoff Vinci 01 von Vincience (Pentadecapeptide-1 Ein weiteres bevorzugtes Aminosäureoligomer ist das Peptidderivat L-Glutamylaminoethyl-indol (Glistin von Exsymol).

Erfindungsgemäß besonders bevorzugt ist die Kombination aus N-Palmitoyl-Gly-His-Lys und N-Palmitoyl-Gly-Gln-Pro-Arg, wie sie beispielsweise in dem Rohstoff Matrixyl 3000 von der Firma Sederma erhältlich ist.

Die Polymere der Aminosäuren und/oder der N-C₂-C₂₄-Acylaminosäuren sind bevorzugt ausgewählt aus pflanzlichen und tierischen Proteinhydrolysaten und/oder Proteinen. Tierische Proteinhydrolysate sind z. B. Elastin-, Collagen-, Keratin-, Seiden-, Conchiolin- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Mandel-, Erbsen-, Kartoffel- und Reisproteinhydrolysate. Besonders bevorzugt sind Sojaproteinhydrolysate, besonders bevorzugt Sojaproteinhydrolysate mit einem mittleren Molekulargewicht im Bereich von 1200 - 1800 Dalton, bevorzugt im Bereich von 1400 - 1700 Dalton, z. B. unter dem Handelsnamen Ridulisse C^{®} von der Firma Silab erhältlich, und Sojaproteinhydrolysate mit einem mittleren Molekulargewicht im Bereich von 600 - 1000 Dalton, bevorzugt 800 Dalton, z. B. unter dem Handelsnamen Phytokine^{®} von Coletica erhältlich. mit Kokosfettsäuren N-acylierten und/oder veresterten Sojaproteinhydrolysaten in Form ihrer Alkalimetallsalze. Kokosfettsäuren umfassen überwiegend Alkancarbonsäuren mit einer Anzahl an Kohlenstoffatomen von 8 - 18, insbesondere Caprylsäure, Caprinsäure, Laurinsäure, Myristinsäure, Palmitinsäure und Stearinsäure. Bevorzugte Alkalimetallsalze sind ausgewählt aus Lithium-, Natrium- und Kaliumsalzen, wobei die Kaliumsalze besonders bevorzugt sind. Ein weiteres, erfindungsgemäß besonders bevorzugtes Sojaproteinhydrolysat ist ein mit Kokosfettsäuren N-acyliertes und/oder verestertes Sojaproteinhydrolysat in Form des Kaliumsalzes, das unter der Handelsbezeichnung Coccopolipeptide di Soja von der Firma Sinerga erhältlich ist. Weiterhin erfindungsgemäß bevorzugt sind Keratinhydrolysate, insbesondere Wollkeratinhydrolysate. Ein besonders bevorzugtes Wollkeratinhydrolysat ist unter der Bezeichnung Keratec Pep von der Firma Croda erhältlich. Keratec Pep weist eine kleinere Molekulargewichtsfraktion mit einem durchschnittlichen Molekulargewicht von 150 Dalton und eine größere Molekulargewichtsfraktion mit einem durchschnittlichen Molekulargewicht von 1265 Dalton auf. Weiterhin erfindungsgemäß bevorzugt sind Conchiolinhydrolysate, insbesondere solche, die unter den Bezeichnungen Pearl Protein Extract und Pearl Protein Extract BG von der Firma Maruzen erhältlich sind. Conchiolin ist ein komplexes Protein, das aus dem äußeren Epithelium von Mollusken, insbesondere von Perlmuscheln und diversen Schneckenarten, erzeugt wird und das durch Einlagerung von Calciumcarbonat-Kristallen die sehr stabile Schale dieser Mollusken bildet. Proteinhydrolysate können naturgemäß auch monomere Aminosäuren und Oligopeptide enthalten; ihre Zusammensetzung ist normalerweise nicht definiert.

Ebenfalls bevorzugt ist der Einsatz von Acylderivaten der Proteinhydrolysate, z. B. in Form ihrer Fettsäure-Kondensationsprodukte.

Ein weiterer erfindungsgemäß bevorzugter optionaler Wirkstoff ist das Peptidderivat L-Glutamylaminoethyl-indol (erhältlich z. B. unter dem Handelsnamen Glistin von Exsymol).

In einer weiteren bevorzugten Ausführungsform sind die Polymeren der Aminosäuren ausgewählt aus DNA-Reparaturenzymen. Erfindungsgemäß bevorzugte DNA-Reparaturenzyme sind Photolyase und T4 Endonuclease V, letztere im Weiteren mit "T4N5" abgekürzt. Diese beiden Enzyme sind im Stand der Technik bereits als sogenannte DNA-Reparatur-Enzyme bekannt. Unter DNA-Reparatur ist definitionsgemäß die Spaltung bzw. Entfernung von UV-induzierten Pyrimidindimeren aus der DNA zu verstehen.

Photolyase ist die Kurzbezeichnung für Desoxyribodipyrimidin-Photolyase bzw. DNA-Photolyase, ein Enzym mit der Klassifizierungsnummer EC 4.1.99.3. Eine besonders effiziente Photolyase stammt aus *Anacystis nidulans,* einem phototrophen marinen Mikroorganismus. Die Photolyase aus *A. nidulans* wird in technisch relevanten Mengen mittlerweile aus E. coli gewonnen. Photolyase ist zur Aktivierung auf Licht angewiesen.

Das Enzym T4 Endonuclease V wird vom *den*V-Gen der Bakteriophage T4 produziert und gehört zu den Phosphodiesterasen, die die Nucleinsäuren an der (5'-3')-Bindung hydrolytisch spalten. T4N5 ist auch ohne Lichteinfluss aktiv.

Erfindungsgemäß besonders bevorzugt ist der Einsatz von liposomenverkapselten DNA-Reparaturenzymen. Liposomenverkapselte Photolyase ist im Handel z. B. unter der Produktbezeichnung Photosome™, liposomenverkapselte T4N5 z. B. unter der Bezeichnung Ultrasome™ von der Firma AGI Dermatics, USA, erhältlich.

Erfindungsgemäß besonders bevorzugte Zusammensetzungen sind **dadurch gekennzeichnet, dass** sie mindestens eines der Handelsprodukte Photosomes™ oder Ultrasomes™ in Gesamtmengen von 0,1 - 10 Gew.-%, bevorzugt 0,5 - 5,0 Gew.-% und besonders bevorzugt 1,0 - 4,0 Gew.-%, bezogen auf die gesamte erfindungsgemäße Zusammensetzung, enthalten.

Erfindungsgemäß besonders bevorzugte Zusammensetzungen sind **dadurch gekennzeichnet, dass** sie mindestens ein Monomer, Oligomer oder Polymer von Aminosäuren, N-C₂-C₂₄-Acyl-aminosäuren und/oder den Estern und/ oder den physiologisch verträglichen Metallsalzen dieser Substanzen in Gesamtmengen von 0,0000001 - 10 Gew.-%, bevorzugt 0,001 - 5 Gew.-% und besonders bevorzugt 0,01 - 1 - 2 - 3 Gew.-%, jeweils bezogen auf den Aktivsubstanzgehalt in der gesamten erfindungsgemäßen Zusammensetzung, enthalten.

In einer weiteren bevorzugten Ausführungsform enthalten die erfindungsgemäßen Zusammensetzungen mindestens ein DNA-Oligonucleotid oder mindestens ein RNA-Oligonucleotid.

Erfindungsgemäß werden unter einem Oligonucleotid Polymerisate aus 2 bis 20, bevorzugt 2 bis 10 Mononucleotiden verstanden, die ebenso wie bei Polynucleotiden und Nucleinsäuren durch Phosphorsäurediester-Brücken verknüpft sind. Die Nucleotide bestehen aus Nucleobasen (meist Pyrimidin- oder Purin-Derivaten), Pentosen (meist D-Ribofuranose oder 2-Desoxy-D-ribofuranose in β-N-glykosidischer Bindung an die Nucleobase) und Phosphorsäure. Die Mononucleotide sind zum Beispiel Adenosinphosphate, Cytidinphosphate, Guanosinphosphate, Uridinphosphate und Thymidinphosphate, insbesondere CMP (Cytidin-5'-monophosphat), UDP (Uridin-5'-diphosphat), ATP (Adenosin-5 '-triphosphat) und GTP (Guanosin-5'-triphosphat).

Ein erfindungsgemäß besonders bevorzugtes Oligonucleotid ist das Thymidin-Dinucleotid.

Erfindungsgemäß besonders bevorzugte Zusammensetzungen sind **dadurch gekennzeichnet, dass** sie mindestens ein DNA-Oligonucleotid und/oder ein RNA-Oligonucleotid in Gesamtmengen von 0,000001 - 5 Gew.-%, bevorzugt 0,0001 - 0,5 Gew.-% und besonders bevorzugt 0,001 - 0,05 Gew.-%, bezogen auf die gesamte Zusammensetzung, enthalten.

In einer weiteren bevorzugten Ausführungsform enthalten die erfindungsgemäßen Zusammensetzungen als optionalen Zusatzstoff mindestens eine natürliche Betainverbindung. Erfindungsgemäße natürliche Betainverbindungen sind natürlich vorkommende Verbindungen mit der Atomgruppierung R₃N⁺-CH2-X-COO⁻ gemäß IUPAC-Regel C-816.1. So genannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain (Me₃N⁺-CH₂-COO⁻) und Carnitin (Me₃N⁺-CH₂-CHOH-CH₂-COO⁻), jeweils mit Me = Methyl und X = C-C-Einfachbindung (im Falle des Betains) oder X = -CHOH-CH₂- (im Falle des Carnitins).

Erfindungsgemäß besonders bevorzugte Zusammensetzungen sind **dadurch gekennzeichnet, dass** sie mindestens eine natürliche Betainverbindung in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.

In einer weiteren bevorzugten Ausführungsform enthalten die erfindungsgemäßen Zusammensetzungen mindestens ein Vitamin, Provitamin oder eine als Vitaminvorstufe bezeichnete Verbindung aus den Vitamingruppen B, C und E und den Estern der vorgenannten Substanzen.

Zur Vitamin B-Gruppe oder zu dem Vitamin B-Komplex gehören unter anderem
- Vitamin B₁, Trivialname Thiamin, chemische Bezeichung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,0005 bis 0,1 - 1 Gew.-%, bezogen auf die gesamte erfindungsgemäße Zusammensetzung, eingesetzt.
- Vitamin B₂, Trivialname Riboflavin, chemische Bezeichung 7,8-Dimethyl-10-(1-D-ribityl)-benzo[g]pteridin-2,4(3*H*,10*H*)-dion. Bevorzugt werden Riboflavin oder seine Derivate in Mengen von 0,0005 bis 0,1 - 1 Gew.-%, bezogen auf die gesamte erfindungsgemäße Zusammensetzung, eingesetzt.
- Vitamin B₃. Unter dieser Bezeichnung werden die Verbindungen Nicotinsäure und Nicotinsäureamid (Niacinamid) geführt. Erfindungsgemäß bevorzugt ist das Nicotinsäureamid, das in den erfindungsgemäßen Zusammensetzungen bevorzugt in Mengen von 0,0005 bis 0,1 - 1 Gew.-%, bezogen auf die gesamte erfindungsgemäße Zusammensetzung, enthalten ist.
- Vitamin B₅ (Pantothensäure und Panthenol). Bevorzugt wird Panthenol eingesetzt. Erfindungsgemäß bevorzugte Derivate des Panthenols sind insbesondere die Ester und Ether des Panthenols sowie kationisch derivatisierte Panthenole. In einer weiteren bevorzugten Ausführungsform der Erfindung werden an Stelle von sowie zusätzlich zu Pantothensäure oder Panthenol auch Derivate des 2-Furanon mit der allgemeinen Strukturformel (VIT-I) eingesetzt.

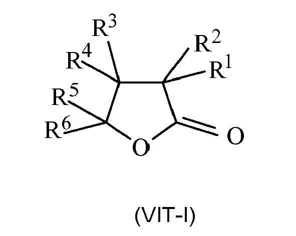

Das erfindungsgemäß außerordentlich bevorzugte 2-Furanon-Derivat ist Pantolacton (Dihydro-3-hydroxy-4,4-dimethyl-2(3H)-furanon), wobei in Formel (I) R¹ für eine Hydroxylgruppe, R² für ein Wasserstoffatom, R³ und R⁴ für eine Methylgruppe und R₅ und R⁶ für ein Wasserstoffatom stehen. Das Stereoisomer (R)-Pantolacton entsteht beim Abbau von Pantothensäure. Erfindungsgemäß besonders bevorzugte Zusammensetzungen sind **dadurch gekennzeichnet, dass** sie mindestens eine der genannten Verbindungen des Vitamin B₅-Typs sowie der 2-Furanonderivate in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.
- Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Erfindungsgemäß besonders bevorzugte Zusammensetzungen sind **dadurch gekennzeichnet, dass** sie mindestens eine Vitamin B₆-Komponente in einer Gesamtmenge von 0,0001 bis 1,0 Gew.-%, insbesondere in Mengen von 0,001 bis 0,01 Gew.-%, enthalten.
- Vitamin B₇ (Biotin), auch als Vitamin H oder "Hautvitamin" bezeichnet. Bei Biotin handelt es sich um (3a*S*,4*S*, 6a*R*)-2-Oxohexahydrothienol[3,4-*d*]-imidazol-4-valeriansäure. Erfindungsgemäß besonders bevorzugte Zusammensetzungen sind dadurch gekennzeichnet, dass sie mindestens eine Komponente, ausgewählt aus Biotin und den Biotinestern, in einer Gesamtmenge von 0,0001 bis 1,0 Gew.-%, insbesondere 0,001 bis 0,01 Gew.-%, enthalten.
- Folsäure (Vitamin B₉, Vitamin B_{c}). Internationaler Freiname für N-[4-(2-Amino-3,4-dihydro-4-oxo-6-pteridinylmethylamino)-benzoyl]-L-glutaminsäure (N-Pteroyl-L-glutaminsäure, PteGlu). Folat wird synonym zu Pteroylglutamat gebraucht, Folate ist der Sammelbegriff für alle Folsäure-wirksamen Verbindungen und bezeichnet eine Substanzklasse, die einen mit 4-Aminobenzoesäure und L-Glutaminsäure verbundenen Pteridin-Ring enthält. Folsäure ist ein Wachstumsfaktor für verschiedene Mikroorganismen und eine Verbindung mit Vitamincharakter, die in der Natur meist als Polyglutamat und in reduzierter Form (7,8-Dihydrofolsäure, H₂Folat, DHF; Tetrahydrofolsäure, H₄Folat, THF; 5'-Methyl-Tetrahydrofolsäure, CH₃-H₄Folat, MeTHF) vorkommt. Erfindungsgemäß besonders bevorzugte Zusammensetzungen sind **dadurch gekennzeichnet, dass** sie mindestens eine Komponente, ausgewählt aus Folsäure, Folaten und deren Estern, in einer Gesamtmenge von 0,0001 bis 1,0 Gew.-%, insbesondere 0,01 bis 0,5 Gew.-%, bezogen auf die Zusammensetzung, enthalten.
- Orotsäure (Vitamin B₁₃, 1,2,3,6-Tetrahydro-2,6-dioxo-4-pyrimidin-carbonsäure, Uracil-6-carbonsäure, Molkensäure). Orotsäure, ihr Cholinester oder Orotsäure-Metallsalze (Orotate von Ca, Cr, Fe, K, Co, Cu, Li, Mg, Mn, Na, Zn, Sn) sind erfindungsgemäß besonders bevorzugt. Erfindungsgemäß besonders bevorzugte Zusammensetzungen sind **dadurch gekennzeichnet, dass** sie mindestens eine Komponente, ausgewählt aus Orotsäure, Orotaten und deren Estern, in einer Gesamtmenge von 0,0001 - 1,0 Gew.-%, insbesondere 0,01 - 0,5 Gew.-%, bezogen auf die Zusammensetzung, enthalten.

In einer weiteren bevorzugten Ausführungsform enthalten die erfindungsgemäßen Zusammensetzungen mindestens eine Substanz, die ausgewählt ist aus den Vitaminen, Provitaminen und Vitaminvorstufen der Gruppe B₁, B₂, B₃, B₆, B₇, B₉, B₁₃ und deren Estern und aus Pantolacton. Bevorzugte Vitamine, Provitamine und Vitaminvorstufen der Gruppe C und deren Ester sind Vitamin C (Ascorbinsäure) und die Derivate Ascorbylpalmitat, -stearat, -dipalmitat, -acetat, Magnesiumascorbylphosphat, Natriumascorbylphosphat, Natrium- und Magnesiumascorbat, Dinatriumascorbylphosphat und -sulfat, Kaliumascorbyltocopherylphosphat, Chitosanascorbat oder Ascorbylglucosid. Die Kombination mit Tocopherolen kann ebenfalls bevorzugt sein. Erfindungsgemäß besonders bevorzugte Zusammensetzungen sind **dadurch gekennzeichnet, dass** sie mindestens eine der genannten Verbindungen des Vitamin C-Typs in einer Gesamtmenge von 0,05 bis 5 Gew.-%, bevorzugt 0,1 bis 3 Gew.-%, besonders bevorzugt 0,5 bis 1 - 2 Gew.-%, jeweils bezogen auf die gesamte Zusammensetzung, enthalten.

Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, -succinat, -linoleat, -oleat, Tocophereth-5, Tocophereth-10, Tocophereth-12, Tocophereth-18, Tocophereth-50 und Tocophersolan. Erfindungsgemäß besonders bevorzugte Zusammensetzungen sind **dadurch gekennzeichnet, dass** sie mindestens eine Substanz, ausgewählt aus Tocopherol und seinen Derivaten, in einer Gesamtmenge von 0,05 bis 5 Gew.-%, bevorzugt 0,1 bis 3 Gew.-%, besonders bevorzugt 0,5 bis 1 - 2 Gew.-%, jeweils bezogen auf die gesamte Zusammensetzung, enthalten.

Pantolacton, Nicotinsäureamid, Pyridoxin, Pyridoxamin, Pyridoxal, Biotin, Ascorbylpalmitat, Ascorbylacetat, Mg-Ascorbylphosphat, Na-Ascorbylphosphat, Natrium- und Magnesiumascorbat und die Tocopherolester, besonders Tocopherylacetat, sind erfindungsgemäß besonders bevorzugt.

In einer weiteren bevorzugten Ausführungsform enthalten die erfindungsgemäßen Zusammensetzungen neben der erfindungsgemäßen Wirkstoffkombination mindestens eine α-Hydroxycarbonsäure, α-Ketocarbonsäure oder β-Hydroxycarbonsäure oder deren Ester-, Lacton- oder Salzform. Erfindungsgemäß bevorzugte α-Hydroxycarbonsäuren oder α-Ketocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methyl-bernsteinsäure, Gluconsäure, Brenztraubensäure, Glucuronsäure und Galacturonsäure. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Erfindungsgemäß besonders bevorzugte Zusammensetzungen sind **dadurch gekennzeichnet, dass** sie neben der erfindungsgemäßen Wirkstoffkombination mindestens eine α-Hydroxycarbonsäure, α-Ketocarbonsäure und/oder β-Hydroxycarbonsäure oder ein Derivat, insbesondere einen Ester, ein Lacton oder ein Salz hiervon, in einer Gesamtmenge von 0,01 - 10 Gew.-%, bevorzugt 0,1 - 5 Gew.-%, besonders bevorzugt 0,5 - 1 - 2 Gew.-%, jeweils bezogen auf die gesamte Zusammensetzung, enthalten.

In einer weiteren bevorzugten Ausführungsform enthalten die erfindungsgemäßen Zusammensetzungen mindestens ein natürlich vorkommendes Xanthin-Derivat, ausgewählt aus Coffein, Theophyllin, Theobromin und Aminophyllin. Erfindungsgemäß bevorzugt sind die natürlich vorkommenden Xanthin-Derivate in Mengen von 0,0001 bis 1 Gew.-%, besonders bevorzugt 0,001 bis 0,5 Gew.-% und außerordentlich bevorzugt 0,005 bis 0,1 Gew.-%, jeweils bezogen auf die gesamte Zusammensetzung, enthalten.

In einer weiteren bevorzugten Ausführungsform enthalten die erfindungsgemäßen Zusammensetzungen Ectoin. Ectoin ist der Trivialname für 2-Methyl-1,4,5,6-tetrahydropyrimidin-4-carboxylat. Erfindungsgemäß bevorzugt ist Ectoin in Mengen von 0,0001 bis 1 Gew.-%, besonders bevorzugt 0,001 bis 0,5 Gew.-% und außerordentlich bevorzugt 0,005 bis 0,01 Gew.-%, jeweils bezogen auf die gesamte Zusammensetzung, enthalten.

In einer weiteren bevorzugten Ausführungsform enthalten die erfindungsgemäßen Zusammensetzungen Kreatin. Kreatin ist der Trivialname für *N*-Methyl-guanidino-essigsäure bzw. N-Amidinosarkosin. Erfindungsgemäß bevorzugt ist Kreatin in Mengen von 0,0001 bis 1 Gew.-%, besonders bevorzugt 0,001 bis 0,5 Gew.-% und außerordentlich bevorzugt 0,01 bis 0,1 Gew.-%, jeweils bezogen auf die gesamte Zusammensetzung, enthalten.

In einer weiteren bevorzugten Ausführungsform enthalten die erfindungsgemäßen Zusammensetzungen mindestens einen Olivenblattextrakt (Olea Europaea (Olive) Leaf Extract). Erfindungsgemäß besonders bevorzugte Zusammensetzungen sind **dadurch gekennzeichnet, dass** sie mindestens einen Olivenblattextrakt in einer Gesamtmenge von 0,01 bis 5 Gew.-%, bevorzugt 0,1 - 3 Gew.-% und besonders bevorzugt 0,5 bis 1 - 2 Gew.-%, jeweils bezogen auf den Extrakt als Handelsprodukt tel quel in der gesamten erfindungsgemäßen Zusammensetzung, enthalten. Olivenblattextrakte können einen hohen Gehalt an Oleanolsäure und/oder Oleanol aufweisen. In einer weiteren bevorzugten Ausführungsform enthalten die erfindungsgemäßen Zusammensetzungen Oleanolsäure, Oleanol und/oder Oleuropein. Erfindungsgemäß besonders bevorzugte Zusammensetzungen sind **dadurch gekennzeichnet, dass** sie Oleanolsäure, Oleanol und/oder Oleuropein in einer Gesamtmenge von 0,00001 bis 2 Gew.-%, bevorzugt 0,001 bis 1 Gew.-% und besonders bevorzugt 0,05 bis 0,1 Gew.-%, jeweils bezogen auf die gesamte erfindungsgemäße Zusammensetzung, enthalten.

In einer weiteren bevorzugten Ausführungsform enthalten die erfindungsgemäßen Zusammensetzungen Ursolsäure. Erfindungsgemäß besonders bevorzugte Zusammensetzungen sind **dadurch gekennzeichnet, dass** sie Ursolsäure in einer Gesamtmenge von 0,00001 bis 2 Gew.-%, bevorzugt 0,001 bis 1 Gew.-% und besonders bevorzugt 0,05 bis 0,1 Gew.-%, jeweils bezogen auf die gesamte erfindungsgemäße Zusammensetzung, enthalten.

In einer weiteren bevorzugten Ausführungsform enthalten die erfindungsgemäßen Zusammensetzungen mindestens ein Derivat von methyliertem Silanol, vorzugsweise mindestens einen Ester von methyliertem Silanol. Bevorzugte Derivate von methyliertem Silanol sind ausgewählt aus:
- sodium mannuronate methylsilanol (Algisium, Exsymol)
- methylsilanol mannuronate (Algisium C®, Exsymol)
- methylsilanol mannuronate Nylon-12 (Algisium C powder®, Exsymol)
- ascorbylmethylsilanol (Ascorbosilane concentrate C®, Exsymol)
- ascorbylmethylsilanol pectinate (Ascorbosilane C®, Exsymol)
- dimethyl oxobenzodioxsilane (DSBC®, Exsymol)
- dimethyl oxobenzodioxasilane Nylon-12 (DSBC powder®, Exsymol)
- sodium hyaluronate dimethylsilanol (DSH®, Exsymol)
- dimethylsilanol hyaluronate (DSHC®, Exsymol)
- methysilanol glycyrrhizinate (Glysinol®, Exsymol)
- methylsilanolhydroxyproline (Hydroxyprolisilane®, Exsymol)
- methylsilanolhydroxyproline aspartate (Hydroxyprolisilane C®, Exsymol)
- sodium lactate methylsilanol (Lasilium®, Exsymol)
- lactoylmethylsilanol elastinate (Lasilium C®, Exsymol)
- dioleyl tocopheryl methylsilanol (Liposiliol C®, Exsymol)
- methylsilanol acetylmethionate (Methiosilane®, Exsymol)
- acetylmethionylmethylsilanol elastinate (Methiosilane C®, Exsymol)
- methylsilanol PEG 7 glyceryl cocoate (Monosiliol®, Exsymol)
- methylsilanol tri PEG 7 glyceryl cocoate (Monosiliol C®, Exsymol)
- methylsilanol elastinate (Proteosilane C®, Exsymol)
- pyrollidone carboxylate caustic methylsilanol (Silhydrate®, Exsymol)
- pyrollidone carboxylate copper methylsilanol (Silhydrate C®, Exsymol)
- methylsilanolcarboxymethyl theophylline (Theophyllisilane®, Exsymol)
- methylsilancarboxymethyl theophylline alginate (Theophyllisilane C® Exsymol)
- methylsilanol acetyltyrosine (Tyrosilane®, Exsymol)
- copper acetyl tyrosinate methylsilanol (Tyrosilane C®, Exsymol).

Besonders bevorzugt sind Sodium Hyaluronate Dimethylsilanol, Dimethylsilanol Hyaluronate, Methylsilanol Mannuronate, Methylsilanol Hydroxyproline und Methylsilanol Hydroxyproline Aspartate. In einer weiteren bevorzugten Ausführungsform enthalten die erfindungsgemäßen Zusammensetzungen mindestens ein Derivat von methyliertem Silanol in Gesamtmengen von 0,001 - 5 Gew.-%, bevorzugt 0,005 - 1 Gew.-% und besonders bevorzugt 0,01 - 0,5 Gew.-%, jeweils bezogen auf die Aktivsubstanz in der gesamten erfindungsgemäßen Zusammensetzung. In einer weiteren bevorzugten Ausführungsform enthalten die erfindungsgemäßen Zusammensetzungen Phytinsäure. Erfindungsgemäß besonders bevorzugte kosmetische oder dermatologische Zusammensetzungen sind **dadurch gekennzeichnet, dass** sie Phytinsäure in einer Gesamtmenge von 0,001 - 1 Gew.-%, bevorzugt 0,01 - 0,5 Gew.-% und besonders bevorzugt 0,05 - 0,1 Gew.-% enthalten, jeweils bezogen auf die gesamte Zusammensetzung.

In einer weiteren bevorzugten Ausführungsform enthalten die erfindungsgemäßen Zusammensetzungen mindestens eine UV-absorbierende Substanz enthalten. Bei einem Einsatz in geringen Mengen (bis etwa 1 Gew.-%) dient die UV-absorbierende Substanz dazu, vorteilhafterweise die Lichtbeständigkeit sonstiger Rezepturbestandteile zu verbessern (Produktschutz). Bei einem Einsatz in höheren Mengen (ab mehr als 1 Gew.-%) dient die UV-absorbierende Substanz dazu, die behandelte Haut vor den schädlichen Auswirkungen der UV-Strahlung zu schützen. Unter UV-absorbierende Substanz sind organische und anorganische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. Verbindungen, die diese gewünschten Eigenschaften aufweisen, sind beispielsweise die durch strahlungslose Desaktivierung wirksamen Verbindungen und Derivate des Benzophenons mit Substituenten in 2- und/oder 4-Stellung. Weiterhin sind auch substituierte Benzotriazole, in 3-Stellung Phenylsubstituierte Acrylate (Zimtsäurederivate), gegebenenfalls mit Cyanogruppen in 2-Stellung, Salicylate sowie Naturstoffe wie Umbelliferon und die körpereigene Urocansäure geeignet. Besondere Bedeutung haben Biphenyl- und vor allem Stilbenderivate, kommerziell als Tinosorb^{®} FD oder Tinosorb^{®} FR ex Ciba erhältlich. Als UV-B-Absorber sind zu nennen 3-Benzylidencampher bzw. 3-Benzylidennorcampher und dessen Derivate, z.B. 3-(4-Methylbenzyliden)campher; 4-Aminobenzoesäurederivate, vorzugsweise 4-(Di-methylamino)benzoesäure-2-ethylhexylester, 4-(Dimethylamino)benzoesäure-2-octylester und 4-(Dimethylamino)benzoesäureamylester; Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3,3-phenylzimtsäure-2-ethylhexylester (Octocrylene); Ester der Salicylsäure, vorzugsweise Salicylsäure-2-ethylhexylester, Salicylsäure-4-isopropylbenzylester, Salicylsäurehomomenthylester; Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon; Ester der Benzalmalonsäure, vorzugsweise 4-Methoxy-benzmalonsäuredi-2-ethylhexylester; Triazinderivate, wie z.B. 2,4,6-Trianilino-(p-carbo-2'-ethyl-1'-hexyloxy)-1,3,5-triazin und Octyl Triazon, oder Dioctyl Butamido Triazone (Uvasorb® HEB); Propan-1,3-dione, wie z.B. 1-(4-tert.-Butylphenyl)-3-(4'methoxyphenyl)propan-1,3-dion; Ketotricyclo(5.2.1.0)decan-Derivate. Weiterhin geeignet sind 2-Phenylbenzimidazol-5-sul-fonsäure und deren Alkali-, Erdalkali-, Ammonium-, Alkylammonium-, Alkanolammonium- und Glucammoniumsalze; Sulfonsäurederivate von Benzophenonen, vorzugsweise 2-Hydroxy-4-methoxybenzophenon-5-sulfon-säure und ihre Salze; Sulfonsäurederivate des 3-Benzylidencamphers, wie z.B. 4-(2-Oxo-3-bornylidenmethyl)benzol-sulfonsäure und 2-Methyl-5-(2-oxo-3-bornyliden)sulfonsäure und deren Salze.

Als typische UV-A-Filter kommen insbesondere Derivate des Benzoylmethans in Frage, wie 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen. Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse, vorzugsweise nanoisierte Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente bereits für hautpflegende und hautschützende Emulsionen und dekorative Kosmetik verwendet. Die Partikel sollten dabei einen mittleren Durchmesser von weniger als 100 nm, vorzugsweise von 5 bis 50 nm und insbesondere von 15 bis 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. Ebenfalls bevorzugt kann mikronisiertes Zinkoxid verwendet werden. Weitere geeignete UV-Lichtschutzfilter sind dem einschlägigen Stand der Technik zu entnehmen.

Die mindestens eine UV-absorbierende Substanz ist in bevorzugten erfindungsgemäßen Zusammensetzungen in einer Gesamtmenge von 0,01 Gew.-% bis 20 Gew.-%, besonders bevorzugt 1 - 10 Gew.-%, außerordentlich bevorzugt 2 Gew.-% bis 7 Gew.-%, enthalten.

Die folgenden Beispiele sollen den Gegenstand der vorliegenden Anmeldung verdeutlichen, ohne ihn hierauf zu beschränken. Alle Mengenangaben in den folgenden Tabellen sind in Gew.-%.

**Tabelle 1: Öl-in-Wasser-Emulsionen**

| | 1 | 2 | 3 | 4 | 5 |
|---|---|---|---|---|---|
| Distelöl | 3,00 | 3,00 | 3,00 | 3,00 | 3,00 |
| Caprylic/Capric Triglyceride | 5,00 | 5,00 | 5,00 | 5,00 | 5,00 |
| Novata AB | 2,00 | 2,00 | 2,00 | 2,00 | 2,00 |
| Lanette 22 | 1,00 | 1,00 | 1,00 | 1,00 | 1,00 |
| Cutina MD | 2,00 | 2,00 | 2,00 | 2,00 | 2,00 |
| Stenol 1618 | 1,00 | 1,00 | 1,00 | 1,00 | 1,00 |
| Baysilon M350 | 1,00 | 1,00 | 1,00 | 1,00 | 1,00 |
| Propylparaben | 0,20 | 0,20 | 0,20 | 0,20 | 0,20 |
| Dry Flo Plus | 1,00 | 2,00 | 3,00 | 2,00 | 3,00 |
| Propylenglycol | 5,00 | 5,00 | 5,00 | 5,00 | 5,00 |
| Glycerin | 5,00 | 3,00 | 3,00 | 3,00 | 3,00 |
| Methylparaben | 0,20 | 0,20 | 0,20 | 0,20 | 0,20 |
| Phenoxyethanol | 0,9 | 0,5 | 0,9 | 0,5 | 0,9 |
| Parfum | 0,10 | 0,10 | 0,10 | 0,10 | 0,10 |
| wässrige Tetrapeptid-Zubereitung Nr. 1 | 4 | 2 | 3 | 2 | 3 |
| Astaxanthin | - | - | - | - | 0,05 |
| Superoxiddismutase | 0,1 | - | - | - | - |
| 3,4-Dihyd ro-7-methoxy-2,2-dimethyl-2H-1-benzopyran-6-ol | - | 0,01 | - | - | - |
| Dihydroquercetin | - | - | 0,5 | - | - |
| pulverisierter Traubenkern-extrakt (Vitis vinifera seed), 95 Gew.-% Polyphenole | - | - | - | 0,5 | - |
| Wasser | ad 100 | ad 100 | ad 100 | ad 100 | ad 100 |

**Tabelle 2: Öl-in-Wasser-Emulsionen**

| | **1** | **2** | **3** | **4** | **5** |
|---|---|---|---|---|---|
| Gehärtetes Sojalecithin | 0,50 | 0,50 | 0,50 | 0,50 | 0,50 |
| Cutina MD | 1,00 | 1,00 | 1,00 | 1,00 | 1,00 |
| Lanette 22 | 2,00 | 2,00 | 2,00 | 2,00 | 2,00 |
| Baysilone M 350 | 0,50 | 0,50 | 0,50 | 0,50 | 0,50 |
| Propylparaben | 0,20 | 0,20 | 0,20 | 0,20 | 0,20 |
| Dow Corning 9040 | 1,00 | 1,00 | 1,00 | 1,00 | 1,00 |
| Glycerin | 4,50 | 3,00 | 3,00 | 3,00 | 4,50 |
| Hexandiol | 6,00 | 3,00 | | 3,00 | 6,00 |
| Methylparaben | 0,20 | 0,20 | 0,20 | 0,20 | 0,20 |
| Tego Carbomer 140 | 0,30 | 0,30 | 0,30 | 0,30 | 0,30 |
| DSH C N | 5,00 | 5,00 | 5,00 | 5,00 | 5,00 |
| Propylenglycol | 5,00 | 5,00 | 5,00 | 5,00 | 5,00 |
| Simulgel EPG | 1,50 | 1,50 | 1,50 | 1,50 | 1,50 |
| TiO₂ (Füllstoff) | 0,50 | 0,50 | 0,50 | 0,50 | 0,50 |
| wässrige Tetrapeptid-Zubereitung Nr. 2 | 4 | 2 | 3 | 2 | 3 |
| Zeaxanthin | 0,1 | - | - | - | - |
| 3,4-Dihydro-7-methoxy-2,2-dimethyl-2H-1-benzopyran-6-ol | - | 0,01 | - | - | - |
| Dihydroquercetin | - | - | 0,5 | - | - |
| Pulverisierter Traubenkernextrakt (Vitis vinifera seed), 95 Gew.-% Polyphenole | - | - | - | 0,5 | - |
| Lycopin | - | - | - | - | 0,07 |
| Wasser | ad 100 | ad 100 | ad 100 | ad 100 | ad 100 |

**Tabelle 3: Öl-in-Wasser-Emulsionen mit Sonnenschutzfaktor**

| | 1 | 2 | 3 | 4 | 5 |
|---|---|---|---|---|---|
| Emulsiphos 677660 | 3 | 3 | 3 | 3 | 3 |
| Dibutyladipat | 5 | 5 | 5 | 5 | 5 |
| Caprylic/Capric Triglyceride | 3,5 | 3,5 | 3,5 | 3,5 | 3,5 |
| Safloröl raffiniert | 2 | 2 | 2 | 2 | 2 |
| Behenylalkohol | 3 | 3 | 3 | 3 | 3 |
| 2-Propylheptyl octanoate | 1 | 1 | 1 | 1 | 1 |
| Bienenwachs | 0,2 | 0,2 | 0,2 | 0,2 | 0,2 |
| COSMEDIA SP | 0,5 | 0,5 | 0,5 | 0,5 | 0,5 |
| Butylmethoxydibenzoylmethan | 3 | 3 | 3 | 3 | 3 |
| Parsol SLX | 3 | 3 | 3 | 3 | 3 |
| Propylparaben | 0,2 | 0,2 | 0,2 | 0,2 | 0,2 |
| Hexandiol-1,6 | 5 | 5 | 5 | 5 | 5 |
| Glycerin 86% | 4,5 | 4,5 | 4,5 | 4,5 | 4,5 |
| Sorbit 70% | 2 | 2 | 2 | 2 | 2 |
| Methylparaben | 0,2 | 0,2 | 0,2 | 0,2 | 0,2 |
| Tego Carbomer 140 | 0,5 | 0,5 | 0,5 | 0,5 | 0,5 |
| Trinatriumnitrilotriacetat | 0,04 | 0,04 | 0,04 | 0,04 | 0,04 |
| Cosmedia Silc | 2 | 2 | 2 | 2 | 2 |
| Aminomethyl propanol (99%) | 0,99 | 0,99 | 0,99 | 0,99 | 0,99 |
| Neo Heliopan Hydro | 3 | 3 | 3 | 3 | 3 |
| Natriumhydroxid Perlen | 0,365 | 0,365 | 0,365 | 0,365 | 0,365 |
| wässrige Tetrapeptid-Zubereitung Nr. 3 | 4 | 2 | 3 | 2 | 3 |
| Superoxiddismutase | 0,1 | - | - | - | - |
| Lutein | - | 0,08 | - | - | - |
| Dihydroquercetin | - | - | 0,5 | - | - |
| Pulverisierter Traubenkernextrakt (Vitis vinifera seed), 95 Gew.-% Polyphenole | - | - | - | 0,5 | - |
| Lycopin | - | - | - | - | 0,1 |
| Wasser, vollentsalzt | ad 100 | ad 100 | ad 100 | ad 100 | ad 100 |

**Tabelle 4: Öl-in-Wasser-Emulsionen**

| | **1** | **2** | **3** | **4** | **5** |
|---|---|---|---|---|---|
| Montanov 202 | 5,0 | 5,0 | 5,0 | 5,0 | 5,0 |
| Cutina MD | 2,0 | 2,0 | 2,0 | 2,0 | 2,0 |
| Cetearyl Alcohol | 1,0 | 1,0 | 1,0 | 1,0 | 1,0 |
| Dibutyladipat | 9,0 | 9,0 | 9,0 | 9,0 | 9,0 |
| Dow Corning 245 | - | 7,0 | 3,0 | - | 7,0 |
| Dow Corning 9040 | 1,0 | 1,0 | - | 1,0 | 1,0 |
| Parfum | 0,3 | 0,3 | 0,3 | 0,3 | 0,3 |
| Fucogel 1000 | - | 2,0 | - | - | 2,0 |
| Keltrol SF | - | 0,2 | 0,2 | - | 0,2 |
| Aristoflex AVC | - | - | 0,5 | - | - |
| Hexandiol-1,6 | 6,0 | 6,0 | - | 6,0 | 6,0 |
| Glycerin | 3,0 | 5,0 | 10,0 | 3,0 | 5,0 |
| Dow Corning 200 Fluid (5 cSt) | 7,0 | 7,0 | - | 7,0 | 7,0 |
| Dow Corning 245 | - | 5,0 | 3,0 | - | 5,0 |
| Dow Corning 9040 | 1,0 | - | 1,0 | 1,0 | - |
| Propylenglycol | 2,0 | 2,0 | - | 2,0 | 2,0 |
| Tego Carbomer 140 | 0,3 | 0,3 | 0,3 | 0,3 | 0,3 |
| Dry Flo Plus | 1,0 | 1,0 | 1,0 | 1,0 | 1,0 |
| wässrige Tetrapeptid-Zubereitung Nr. 4 | 4 | 2 | 3 | 2 | 3 |
| Astaxanthin | 0,1 | - | - | - | - |
| Zeaxanthin | - | 0,07 | - | - | - |
| Lycopin | - | - | 0,05 | - | - |
| Lutein | - | - | - | 0,03 | - |
| Coenzym Q-10 | - | - | - | - | 0,05 |
| Wasser | ad 100 | ad 100 | ad 100 | ad 100 | ad 100 |

Die wässrige Tetrapeptid-Zubereitung Nr. 1 enthält in Sojalecithin-Liposomen verkapseltes Tetrapeptid Lysin-Asparaginsäure-Isoleucin-Citrullin und hat folgende Zusammensetzung:
0,01 Gew.-% Lysin-Asparaginsäure-Isoleucin-Citrullin (Lys-Asp-Ile-Cit-NH₂)
5 Gew.-% Lysinhydrochlorid
4 Gew.-% Sojalecithin
ad 100 Gew.-% Wasser

Die wässrige Tetrapeptid-Zubereitung Nr. 2 enthält unverkapseltes Tetrapeptid Lysin-Asparaginsäure-Isoleucin-Citrullin und hat folgende Zusammensetzung:
0,01 Gew.-% Lysin-Asparaginsäure-Isoleucin-Citrullin (Lys-Asp-Ile-Cit-NH₂)
5 Gew.-% Lysinhydrochlorid
ad 100 Gew.-% Wasser

Die wässrige Tetrapeptid-Zubereitung Nr. 3 enthält in Sojalecithin-Liposomen verkapseltes Tetrapeptid Lysin-Asparaginsäure-Isoleucin-Citrullin und hat folgende Zusammensetzung:
0,01 Gew.-% Lysin-Asparaginsäure-Isoleucin-Citrullin (Lys-Asp-Ile-Cit-NH₂)
4 Gew.-% Sojalecithin
ad 100 Gew.-% Wasser

Die wässrige Tetrapeptid-Zubereitung Nr. 4 enthält unverkapseltes acyliertes Tetrapeptid N-Palmitoyl-Lysin-Asparaginsäure-Isoleucin-Citrullin und hat folgende Zusammensetzung:
0,01 Gew.-% N-Palmitoyl-Lysin-Asparaginsäure-Isoleucin-Citrullin
5 Gew.-% Lysinhydrochlorid
60 Gew.-% Glycerin
10 Gew.-% 1,3-Butylenglycol
0,2 Gew.-% Coco Glucoside
ad 100 Gew.-% Wasser

Liste der verwendeten Rohstoffe

| Handelsname | INCI-Bezeichnung | Lieferant/Herstel ler |
|---|---|---|
| Aristoflex AVC | Ammonium Acryloyldimethyl Taurate/VP Copolymer/, t-Butyl Alcohol | Clariant |
| Baysilon M 350 | Dimethicone (350 cSt) | GE Bayer Silicones |
| Cosmedia Silc | Silica | Cognis |
| Cosmedia SP | Sodium Polyacrylate | Cognis |
| Cutina MD | Glyceryl Stearate (Mischung aus Glycerylmono-und distearat) | Cognis |
| Dow Corning 9040 | Cyclomethicone/ Dimethicone Crosspolymer (87-88%/12-13%) | Dow Corning |
| Dow Corning^{®}200 Fluid, 5 cSt. | Dimethicone | Dow Corning |
| Dow Corning^{®}245 Fluid | Cyclomethicone | Dow Corning |
| Dry Flo Plus | Aluminium Starch Octenylsuccinate | National Starch |
| DSH C N | Water, Dimethylsilanol Hyaluronate | Exsymol |
| Emulsiphos 677660 | Potassium Cetyl Phosphate, Hydrogenated Palm Glycerides | Symrise |
| Fucogel 1000 | Aqua, Biosaccharide Gum-1 (1,1 Gew.-%) | Solabia |
| Keltrol SF | Xanthan Gum | Kelco |
| Lanette^{®} 22 | Behenyl Alcohol | Cognis |
| Montanov 202 | Arachidyl Alkohol, Behenyl Alcohol, Arachidyl Glucoside | Seppic |
| Neo Heliopan Hydro | Phenylbenzimidazol sulfonic acid | Symrise |
| Novata AB | Cocoglycerides | Cognis |
| Parsol SLX | Polysilicone-15 | DSM |
| Simulgel EPG | Aqua (Water)/Sodium Acrylate / Sodium Acryloyldimethyl Taurate Copolymer / Polyisobutene / Caprylic/Capryl Glucoside | Seppic |
| Stenol 16/18 | Cetearyl alcohol | Cognis |
| Tego Carbomer 140 | Carbomer | Degussa |

## Patentansprüche

1. Kosmetische Zusammensetzung, enthaltend das Tetrapeptid Lysin-Asparaginsäure-Isoleucin-Citrullin Lys-Asp-Ile-Cit-NH₂ und mindestens ein Antioxidans, das ausgewählt ist aus
- Carotinoiden, insbesondere Astaxanthin und Lycopin,
- Dihydroquercetin,
- 3,4-Dihydro-7-methoxy-2,2-dimethyl-2H-1-benzopyran-6-ol,
- Vitis vinifera-Polyphenolen,
- Ubichinonen und/oder Ubichinolen,
- Superoxid-Dismutasen,
- Ellagsäure und/oder Ellagaten,
- sowie Mischungen dieser Antioxidantien.

2. Kosmetische Zusammensetzung gemäß Anspruch 1, **dadurch gekennzeichnet, dass** das Tetrapeptid Lysin-Asparaginsäure-Isoleucin-Citrullin Lys-Asp-Ile-Cit-NH₂ in einer Menge von 0,00001 - 0,001 Gew.-%, bevorzugt 0,0001 - 0,0008 Gew.-%, besonders bevorzugt 0,0002 - 0,0006 Gew.-%, außerordentlich bevorzugt 0,0003 - 0,0004 Gew.-%, enthalten ist, jeweils bezogen auf das Gesamtgewicht der kosmetischen Zusammensetzung.

3. Kosmetische Zusammensetzung gemäß Anspruch 1 oder 2, **dadurch gekennzeichnet, dass** das mindestens eine Antioxidans in einer Gesamtmenge von 0,001 - 2 Gew.-%, bevorzugt 0,005 - 1 Gew.-%, besonders bevorzugt 0,01 - 0,5 Gew.-%, außerordentlich bevorzugt 0,05 - 0,2 Gew.-%, enthalten ist, jeweils bezogen auf das Gesamtgewicht der kosmetischen Zusammensetzung.

4. Kosmetische Zusammensetzung gemäß Anspruch 1, 2 oder 3, **dadurch gekennzeichnet, dass** das Tetrapeptid Lysin-Asparaginsäure-Isoleucin-Citrullin Lys-Asp-Ile-Cit-NH₂ und die Gesamtmenge an mindestens einem Antioxidationsmittel, das ausgewählt ist aus
- Carotinoiden, insbesondere Lycopin und Astaxanthin,
- Dihydroquercetin,
- 3,4-Dihydro-7-methoxy-2,2-dimethyl-2H-1-benzopyran-6-ol,
- Vitis vinifera-Polyphenolen,
- Ubichinonen und/oder Ubichinolen,
- Superoxid-Dismutasen und
- Ellagsäure und/oder Ellagaten,
- sowie Mischungen dieser Antioxidantien,
in einem Gewichtsverhältnis von 1:10 bis 1:1000, bevorzugt 1:50 - 1:500, besonders bevorzugt 1:80 - 1:400, außerordentlich bevorzugt 1:100 - 1:200, enthalten sind.

5. Kosmetische Zusammensetzung gemäß Anspruch 1, 2, 3 oder 4, **dadurch gekennzeichnet, dass** das Tetrapeptid Lysin-Asparaginsäure-Isoleucin-Citrullin Lys-Asp-Ile-Cit-NH₂ am Lysin-Rest mit einem C₂ - C₂₄-Acylrest N-acyliert ist.

6. Kosmetische Zusammensetzung gemäß Anspruch 1, 2, 3, 4 oder 5, **dadurch gekennzeichnet, dass** weiterhin mindestens eine basische Aminosäure, ausgewählt aus Lysin, Arginin und Histidin, sowie Mischungen dieser basischen Aminosäuren, enthalten ist.

7. Kosmetische Zusammensetzung gemäß Anspruch 1, 2, 3, 4, 5 oder 6, **dadurch gekennzeichnet, dass** das Tetrapeptid Lysin-Asparaginsäure-Isoleucin-Citrullin Lys-Asp-Ile-Cit-NH₂ und die Gesamtmenge an mindestens einer basischen Aminosäure, die ausgewählt ist aus Lysin, Arginin und Histidin, in einem Gewichtsverhältnis von 1:100 bis 1:1000, bevorzugt 1:200 - 1:700, besonders bevorzugt 1:300 - 1:600, außerordentlich bevorzugt 1:400 - 1:500, enthalten sind.

8. Kosmetische Zusammensetzung gemäß Anspruch 1, 2, 3, 4, 5, 6 oder 7, **dadurch gekennzeichnet, dass** das Tetrapeptid Lysin-Asparaginsäure-Isoleucin-Citrullin Lys-Asp-Ile-Cit-NH₂ in verkapselter Form, insbesondere in Vesikeln oder Liposomen verkapselt, vorliegt.

9. Kosmetische Zusammensetzung gemäß Anspruch 1, 2, 3, 4, 5, 6, 7 oder 8, **dadurch gekennzeichnet, dass** sie in Form einer Öl-in-Wasser-Emulsion vorliegt.
